# EUROPEAN PATENT SPECIFICATION

(11) **EP 1 660 679 B1**
(45) Date of publication and mention of the grant of the patent: **14.11.2012**
(21) Application number: 04779622.2
(22) Date of filing: 30.07.2004
(51) Int. Cl.: C12Q 1/68, C07H 21/02, C07H 21/04

(54) **SELF-HYBRIDIZING MULTIPLE TARGET NUCLEIC ACID PROBES AND METHODS OF USE**
SELBSTHYBRIDISIERENDE MEHRFACHZIEL-NUKLEINSÄURESONDEN UND VERWENDUNGSVERFAHREN
SONDES A ACIDES NUCLEIQUES CIBLES MULTIPLES A AUTO-HYBRIDATION ET PROCEDES D'UTILISATION

(30) Priority: 01.08.2003 US 491901 P
(43) Date of publication of application: 31.05.2006
(73) Proprietor: Dynal Biotech Inc., Brown Deer, Wisconsin 53223 (US)
(72) Inventor: RAMON, Daniel, S., Brown Deer, Wisconsin 53209 (US); WANG, Lu, Shorewood, Wisconsin 53211 (US)
(74) Representative: Weber, Birgit
(86) International application number: PCT/US2004/024619
(87) International publication number: WO 2005/012548

(56) References cited:
- EP-A- 0 552 931
- WO-A-02/06531
- WO-A-95/13399
- WO-A1-99/66071
- US-A- 5 168 053
- US-A- 5 925 517
- US-A1- 2002 102 557
- US-B1- 6 183 960
- WHITCOMBE D ET AL: "Detection of PCR products using self-probing amplicons and fluorescence", NATURE BIOTECHNOLOGY, NATURE PUBLISHING GROUP, NEW YORK, NY, US LNKD- DOI:10.1038/11751, vol. 17, no. 8, 1 August 1999 (1999-08-01) , pages 804-807, XP002226672, ISSN: 1087-0156

## Description

### FIELD OF THE INVENTION

The present invention relates a method for detecting more than one target sequence, kits and arrays for carrying out said methods. More specifically, the present invention uses nucleic acid probes having multiple probing regions and self-complementary or self-annealing sequences.

### BACKGROUND OF THE INVENTION

The detection of specific nucleic acids is an important tool for diagnostic medicine and molecular biology research. Nucleic acid identification currently plays an important role in identifying infectious organisms such as bacteria and viruses, in probing the expression of normal genes and identifying mutant genes such as oncogenes, in typing tissue for compatibility preceding tissue transplantation, in matching tissue or blood samples for forensic medicine and for exploring homology among genes from different species.

The "gold standard" for determining the identity of a nucleic acid is sequencing of the nucleic acid of interest. Although there are several different methods for directly sequencing nucleic acids, many of these methods remain cumbersome and take an extended period of time to obtain the nucleic acid sequence.

Due to the time and expense involved, alternatives to direct sequencing, often using various nucleic acid probes and strategies, have been developed. However, these strategies have often been found to be unsatisfactory because they are not capable of resolving closely related sequences or ambiguities that arise from different combinations of alleles.

One major focus of tissue typing, paternity testing and disease association has been on the human leukocyte antigen (HLA) gene. The HLA alleles are the most diverse antigenic system in the human genome and encode literally hundreds of alleles that fall into several distinct subgroups or subfamilies. However, standard techniques for DNA typing have often proven inadequate in resolving many of these important alleles. Not only are techniques that are capable of unlocking this genetic diversity rare, such as sequencing analysis, they are also expensive and time consuming.

WO99/66071 describes a Scorpions primer which is used to detect primer extension products by the presence of a detectable change in a signalling system upon production of a primer extension product.

One significant obstacle in HLA typing is the ambiguous combination that originates from the nature of the origin of polymorphisms by genetic recombination. The inability to resolve cis/trans sequence cluster ambiguities in heterozygotes is a major limitation in techniques like using sequence specific oligonucleotide probes of sequencing base type. One solution to this problem is the separation of each particular allele by cloning techniques. However, this is impractical when dealing with a high volume of samples. Other approaches, such as increasing the number of specific amplifications, also have disadvantages because they often still fail to resolve intra-group ambiguities.

Accordingly there remains a need for methods and probes that can detect and identify specific nucleic acids, especially closely related sequences.

### SUMMARY OF THE INVENTION

One embodiment of the present invention provides a method for detecting more than one target sequence on a target nucleic acid comprising:
(a) contacting a nucleic acid probe comprising
   (i) a first probe region specific for a first target sequence, and
   (ii) a second probe region specific for a second target sequence, wherein the first probe region and the second probe region are non-contiguous, the first target sequence and the second target sequence are non-contiguous, or both the first probe region and the second probe region and the first target sequence and the second target sequence are non-contiguous, and wherein the first target sequence and the second target sequence are each an HLA allele; and
   (iii) self-complementary sequences;
   wherein in the presence of the target sequences, hybridisation between the first and second probe regions and the first and second target sequences competes with hybridisation of the self-complementary regions;
   with a sample suspected of containing the target nucleic acid; and
(b) detecting whether one or more of the first probe region and the second probe region binds their respective target sequence, the method optionally comprising the further step of:
(c) amplifying the nucleic acids in the sample suspected of containing the target nucleic acid prior to (a).

For the nucleic acid probe the first target sequence and the second target sequence can be on the same target nucleic acid or different target nucleic acids. In these and other embodiments the probes can be capable of distinguishing the target nucleic acid which comprises both the first target sequence and the second target sequence from nucleic acids that do not comprise both the first target sequence and the second target sequence. In some embodiments there can be two or four self-complementary sequences. In further embodiments, neither the first probe region nor the second probe region comprise at least part of the self-complementary sequences. Similarly, none of the self-complementary sequences can be located between the first probe region and the second probe region.

In some of the present probes at least one of the first probe region and the second probe region includes at least part of one of the self-complementary sequences. In some of these embodiments only one of the first probe region and the second probe region comprises one of the self-complementary sequences. In some probes the first probe region includes at least part of one of the self-complementary sequences and the second probe region includes at least part of another of the self-complementary sequences. In some probes, the self-complementary sequence of the first probe region is at least partially complementary to the self-complementary sequence of the second probe region. In further probes, the self-complementary sequence of the first probe region is not complementary to the second probe region and the self complementary sequence of the second probe region is not complementary to the first probe region.

The present probes can also optionally include a spacer moiety that separates the first probe region and the second probe region. The spacer moiety can be made up partially or wholly of one or more nucleic acids. The nucleic acid probes can also optionally contain one or more arm sequences that make up part one or more of the self-complementary sequences, wherein the one or more arm sequences do not include the probe regions. In these probes one or more self-complementary sequences of the one or more arm sequences are complementary with a self-complementary sequence located at least partially within one of the probe regions. In some of the probes a first self-complementary sequence of a first of the one or more arm sequences is complementary with a second self-complementary sequence of a second of the one or more arm sequences. In some probes at least one of the one or more self- complementary sequences of the one or more arm sequences is complementary to at least a portion of the spacer moiety,

The probes can also include one or more detectable labels which can be the same or different. The labels can be bound to a terminus of the nucleic acid probe or covalently bound to a nucleotide in the nucleic acid probe. In some probes the detectable label is covalently bound to the spacer moiety of the nucleic acid probe. The detectable label can include a linker group, such as an amino linker, which is covalently bound to the nucleic acid probe. Examples of labels include fluorescent molecules, luminescent molecules, solid surfaces, supports, beads, radioactive moieties, and the like.

In the present probes the first probe region and second probe region can have different sequences or the same sequence. For some probes the first target sequence and second target sequence are motifs and the target nucleic acid is an allele such that the first target sequence and second target sequence are motifs on the same allele. Some of the probes do not include a fluorescent quencher moiety.

The present probes can also be provided in an array format located in defined, distinct locations on a solid support. Each defined, distinct area of the array will typically have a plurality of probes that are the same. In some arrays one or more of the plurality of nucleic acid probes is attached the solid support or contained within a well of the solid support. In further arrays the nucleic acid probes in different defined, distinct locations on the solid have one or more different probe regions, one or more of the same probe regions or all of the same probe regions. Some arrays are provided on glass or plastic supports or multi-well plates.

The present invention also provides nucleic acid probes, wherein the probe comprises:
(a) a first probe region specific for a first target sequence;
(b) a second probe region specific for a second target sequence, the first probe region and the second probe region being non-contiguous; and
(c) self-complementary sequences; wherein
in the absence of one or both target sequences(s), the self-complementary sequences of the nucleic acid probe anneal so that the nucleic acid probe adopts a closed configuration, and in the present of one or both target sequence(s) hybridization of the nucleic acid probe with one or both target sequence(s) is favoured such that the probe adopts an open configuration.

Preferably, one or both probe regions are at least partially contained within a loop when the nucleic acid probe is in a closed configuration to prevent hybridization with non-target sequences.

More preferably the nucleic acid probe is capable of distinguishing between allele combinations AB/--, AB/A-, AB/-B, --/A, --/B, A-/-B wherein A and B denote different alleles.

More preferably, one or both target sequences are class 1-HLA-A, HLA-B, HLA-C, HLA-E, HLA-F, and HLA-G, or Class 11-HLA-DRA, HLA-DRB1, HLA-DRB2-9, HLA-DQA1, HLA-DOB1, HLA-DPA1, HLA-DPB1, HLA-DMA, HLA-DMB, HLA-DOA and HLA-DOB.

The present invention also provides kits containing the probes.

Further objects, features and advantages of the invention will be apparent from the following detailed description.

### BRIEF DESCRIPTION OF THE DRAWINGS

FIG. 1 depicts a probe configuration according to the present invention.
FIG. 2 depicts an alternative probe configuration according to the present invention.
FIG.3 depicts a probe configuration according to the present invention where two loops are formed by the self annealing structures.
FIG. 4 depicts a probe configuration according to the present invention where the probe sequences encompass the self annealing sequences.
FIG. 5 depicts a probe configuration according to the present invention where the probe sequences do not encompass the self annealing sequences.

### DETAILED DESCRIPTION

The present invention relates to methods, arrays and kits using or comprising nucleic acid probes that may be used in a variety of manners but which are particularly useful for detecting the presence of particular target nucleotide sequences. Nucleic acid probes are also provided. The nucleic acid probes that contain two or more probe regions, e.g. a first probe region that is specific for a first target sequence and a second probe region that is specific for a second target sequence, as well as self-complementary regions. The configuration of the probing regions is not particularly limited. Generally, the first probe region will be upstream of the second probe region, e.g. 5' of the second probe region in a nucleic acid, which is then downstream of the first probe region, e.g. 3' of the first probe region. The probe regions can hybridize to their respective target sequences independently of one another. The first probe region and the second probe region can be contiguous, provided that the target sequences for the probe regions are not contiguous, or they can be separated by a spacer. In some embodiments, the probe regions and the target regions for which they are specific will be non-contiguous. The probe regions can also be specific for target sequences on a single nucleic acid or they can target sequences on different target nucleic acids. As used herein, a target nucleic acid is a nucleic acid that contains one or more target sequences. Accordingly, there can be a single or multiple target nucleic acids for each nucleic acid probe. When two or more target sequences are on the same nucleic acid a cooperative increase in hybridization occurs between the probe regions and target sequences. The length of the probe regions is not particularly limited as long as the probe regions are specific for the respective target sequences. In practice, the probe regions should bind the target sequence(s) when present whereas the self-annealing sequences of the probe should be annealed together when the target sequence(s) are absent. In some instances the length of the probing regions will be the same, where in other instances they will be different. As a general guideline, the probe regions can be from 7 to 30 or more, such as 9-21 or 12-16, nucleotides in length. However one of skill in the art will recognize that probing regions of different nucleotide lengths are contemplated.

The probe region(s) can be designed to bind to the target sequence(s) in any suitable configuration. Typically this will involve hybridization of nucleic acids in sense-antisense configuration, i.e. the 5' end of one nucleic acid strand is paired with the 3' end of the complementary nucleic acid. The present probes also contemplate atypical binding configurations, including those where the 5' or 3' "ends" of the sequences are internal to, i.e. located in the center section of, the probe as opposed to being closer to the termini of the probe. Each probe sequence can be complementary to its target sequence from 5' to 3' or 3' to 5'. Such arrangements are described in U.S. Patent No. 5,914,230, with particular reference made to the figures. The present probes can have probing regions that are the same sequence or different sequences. In the case where the probing regions are the same, the nucleic acid probe can be used to detect repeated sequences, such as tandem repeats. In some embodiments, the nucleic acid probe can contain third, fourth, fifth, sixth, etc. probing regions that all have the same sequence so that the repeats can be detected and/or quantified. The probe may also contain this number of probing regions having different sequences.

As will be apparent to the skilled artisan the identity of the probing regions will be dictated by the target nucleic acid sequence that is desired to be detected. Accordingly, the probe sequences can have a sense or antisense configuration. When probe regions are complementary to target sequences in different target molecules, each probe region can be complementary to either a sense or antisense target sequence, irrespective of orientation of other probe regions. In addition, orientation of probe regions can be 5'-3' in tandem to 5'-3' or 5'-3' in tandem to 3'-5'. The target nucleic acids for which the probes are specific are not particularly limited. When the target nucleic acids are on the same nucleic acid, the target nucleic acids can be contiguous, or can be separated from one another by one or more, for example 2-5, up to 300 or more, nucleotides. In a similar manner, the source for the target nucleic acids is not particularly limited and, for example, can be obtained from an individual of interest, synthetically or from a depository. In some examples, the target sequences are specific motifs. These motifs can be on the same allele such that the first target sequence and second target sequence are motifs on the same allele.

Although the probe regions are generally made up of nucleic acid sequences, the probe regions can be any suitable moiety or molecule that is specific for any given nucleotide sequence or feature, such as an unmatched base, a mismatched base pair or alternative nucleic acid structure. Accordingly, it is contemplated that the present probes can contain a probing region made up of a protein, peptide, antibody or functional fragment thereof, that binds to a specific sequence or feature.

Self-complementary sequences are sequences contained within a single molecule or nucleic acid ("self" aspect) that are complementary to each other so that they are capable of binding each other under the appropriate hybridization conditions, for example below the melting temperature of the complementary sequences and/or in the absence of the target sequences. The self-complementary regions of the present probes allow different sections of the probe to anneal to each other under certain conditions, such as at temperatures below the melting temperature of the self-complementary sequences or in the absence of the target sequences. Generally, the probes will contain an even number of self-complementary sequences, such as two, four or more because they pair with one another, although the probes can also contain an odd number of complementary sequences where one of the self-complementary sequences is complementary to two other self-complementary sequences. The number and location of the self-complementary sequences within the probe is not particularly limited. For example, one or more of the self-complementary sequences can be contained partially or wholly within the probe regions, partially or wholly within a spacer separating the probe regions, partially or wholly within distal arm regions of the probe, or various combinations of these embodiments, as desired. In some embodiments, probe regions do not contain part of the self-complementary sequences. In further embodiments, at most one of the probe regions will at least partly contain one of the self-complementary sequences. In some probes, only one of the first probe region and the second probe region comprises one of the self-complementary sequences. In this embodiment, the other self-complementary sequence(s) will be located outside of either of the probe regions. Similarly, the self-complementary sequences can be excluded from any or all of the spacers separating the probe regions. In some embodiments, the self-complementary sequences are 3, 4, 5, 6, 7, 8 or 9 nucleotides in length.

The pairing arrangements between the self-complementary sequences is not limited. Accordingly, one self-complementary sequence in a probe, spacer or arm region can be complementary to, and potentially anneal with, a second self-complementary region that is in a probe, spacer or arm region. Examples of such complement configurations include, probe-probe, probe-spacer, probe-arm, spacer-spacer, arm-arm, combinations of these and their inverse configurations. The self-complementary sequences can also span across, and be partially contained within, two different parts of the probe. The self-complementary sequences can alternatively be contained wholly within a single part of the probe. When a self-complementary sequence is contained wholly within one part of the probe, e.g. the probe region, spacer or arm, the self-complementary sequence can make up all or only a part of that part of the probe.

As will be apparent to the skilled artisan, the self-complementary sequences of the present probes should not pair together so strongly that the probe region(s) cannot bind to the target sequence(s) when they are present. One way to avoid this complication is by designing the self-complementary sequences to have a lower melting temperature and/or annealing affinity relative to the melting temperature and/or annealing affinity of the probe region-target sequence. One skilled in the art will recognize that these parameters can be changed as desired, such as by changing the length of the complementary sequences and/or the G/C content of the self-complementary sequences. In some embodiments, the length, sequence, and GC content of the self-complementary sequences is designed such that the melting temperature of the self-complementary sequences is 7-10 °C higher than the temperature at which the probe is used to bind to the target sequence. This design helps to ensure that the self-complementary probes remain annealed in the absence of the target sequence(s). Since the self-complementary regions anneal by an intramolecular hybridization event, their melting temperature typically cannot be precisely predicted by the percent-GC rule. Instead, a DNA folding program (such as the Zuker folding program that is available on the internet, at http://bioinfo.math.rpi.edu/~mfold/dna/form1.cgi) should be utilized to estimate the free energy of formation of the annealed self-complementary sequences, from which a melting temperature can be predicted.

Where the probe regions are specific for more than one target sequence on a single nucleic acid then the nucleic acid probe can be capable of distinguishing the target nucleic acid that comprises both the first target sequence and the second target sequence from nucleic acids that do not comprise both the first target sequence and the second target sequence. As will be understood by the skilled artisan, nucleic acids that do not include both target sequences can contain one or no target sequence. This nucleic acid probe configuration helps to distinguish cis/trans ambiguities in heterozygotes.

The nucleic acid probe can contain one or more spacer moieties located between any of the probe regions and/or arm regions, although the presence of the spacer is not required. The presence of the spacer(s) will render the probe regions non-contiguous. As will be understood by the skilled artisan the spacer moiety should not have a sequence that is complementary to the region of the target nucleic acid located between the first target sequence and the second target sequence. Preferably, the spacer does not interact or interfere with the target nucleic acid or other nucleic acids in the sample. The spacer can be any suitable atom or chain of atoms, and thus its chemical identity is not particularly limited. In some embodiments, the spacer will be made up of nucleotides, such as a homopolynucleotide sequence. In some embodiments, the spacer region can be some other flexible chemical structure, such as a substituted or unsubstituted alkyl or aryl group having an appropriate number of carbon atoms, ribose or 1', 2'-dideoxyribose chains. The spacer can also be a branched or unbranched carbon or heteroatom containing chain, such as a chain containing carbon, oxygen, sulfur and/or nitrogen. The spacer can also contain peptides or be a peptide chain. Peptides can be naturally occurring or non-naturally occurring, for example D-amino acids, as desired. When the spacer is a nucleotide sequence, the spacer can be 1 to 20 nucleotides, or more, long. In some embodiments, when the spacer is located between the probing region and an arm, the spacer can be about 10-20, such as 12-18 or 14-16, nucleotides in length. In other examples, the spacer is located between the probing regions and is about 3-9, such as 5, nucleotides in length. Spacer regions can also be included between the arm sequences and probe regions to help prevent interference between their respective binding events.

The present probes can further contain one or more arm sequences, although these are also not required. When present, the arm sequence(s) will be made up of one or more of the self-complementary sequences but will not be part of the probe regions. The arm sequence(s) are generally located on the end(s) of the probe, e.g. they are not located between the probe regions. In some embodiments, the present nucleic acid probes contain only a single arm sequence, which generally will be complementary to a portion of the spacer region and/or one of the probe regions that can be either a more proximal or distal probe region relative to the arm sequence. In other embodiments, the probe will contain two arm sequences that can be at least partially complementary to one another or complementary with a portion of the spacer region(s) and/or one of the probe regions. In other embodiments, part or all of one or more of the self-complementary sequences located within the arm portions of the nucleic acid probe can be complementary to part or all of the spacer moiety. As such, the entirety of one or more arm regions can be complementary to part of the spacer moiety, but this need not be the case. Similarly, part of the one or more arm regions can be complementary to the entirety of the spacer moiety, but this also is not required. In some embodiments, the entirety of one of the arms can be complementary to the entirety of the spacer moiety. The length of the arm sequences of the probes is only limited in that the probes containing the arm sequences must still be functional. Generally, the arm sequence(s) of the probes will be four, five, six or seven nucleotides in length. GC-rich arms of 5 base pairs will melt between 55 and 60°C, GC-rich arms of 6 base pairs will melt between 60 and 65°C, and GC-rich arms of 7 base pairs will melt between 65 and 70°C.

Reference to arm sequences herein is similar to arm or stem sequences as used in molecular beacon technology. See, e.g., www.molecular-beacons.org; Bonnet et al., Proc Natl Acad Sci USA 96, pp. 6171-6176, 1999; Marras et al., Genet Anal 14, pp. 151-156, 1999; Marras et al., Nucleic Acids Res 30, p. E122, 2002; Mullah et al., Nucleosides & Nucleotides 18, pp. 1311-1312, 1999; Ortiz et al., Mol Cell Probes 12, pp. 219-226, 1998; Tyagi et al., Nat Biotechnol 18, pp. 1191-1196, 2000; Tyagi et al., Nat Biotechnol 16, pp. 49-53, 1998; Tsourkas et al., Nucleic Acids Res 31 (4), p. 1319, 2003; Tsourkas et al., Nucleic Acids Res 30 (19), p. 4208, 2002; Broude et al., Nucleic Acids Res 29 (19), p. e92, 2001; and Tyagi et al., Nat Biotechnol 14, pp. 303-308, 1996. Molecular beacons are also described in U.S. Patent Nos. 5,925,517; 6,037,130; 6,103,476; and 6,150,097. The present probes can be used in the assays and methods described in these patents and publications.

One embodiment of the present probes encompasses molecular beacons when the distal ends of the probe are attached to one or more fluorophores and one or more quencher moieties. However, some of the present probes exclude molecular beacons because they do not contain a fluorescent and/or quencher moiety covalently attached to the probe. Because some of the present probes lack quenchers and/or fluorophores, they are not required to form hairpin structures in the absence of target sequence(s) in contrast to molecular beacons. Other differences between some of the present probes and molecular beacons include:
1. The present probes can resolve homogeneous and non-homogeneous systems;
2. The present probes can be linked to a solid phase, such as beads;
3. The loops of the present probes can include probing sequence(s) and can bind with the stem arm or not;
4. Any arm sequence can bind a probing sequence;
5. An arm sequence can hybridize with any position and is not limited to binding with other termini, e.g. no quencher-fluorophore interaction is required;
6. The loop of the present probe can contain one target sequence or two or more; and/or
7. The present probes can have multiple loops when self-hybridized.

The present probes can also carry one or more detectable labels. For example, the detectable label can be covalently bound to the nucleic acid probe, such as at the terminus of the probe. The detectable label can additionally or alternatively be covalently bound to any nucleotide in the nucleic acid probe, including those in the arm sequences, probe regions or spacer regions. As will be recognized by the skilled artisan, the location of the detectable label moiety in the nucleic acid probe is not particularly limited although the label should not interfere with the binding of the probe regions to their target sequences, or such interference should be kept to a minimum. In some embodiments, the detectable label comprises a linker group, such as an amino linker, which is covalently bound to the nucleic acid probe. The label does not need to be covalently the bound to the probe but should be associated with the probe such that it can help signal the presence or absence of target binding.

When present, the detectable moiety of the present disclosure is not particularly limited. Suitable examples of detectable labels include fluorescent molecules, beads, polymeric beads and fluorescent polymeric beads. Polymeric beads can be made of any suitable polymer including latex or polystyrene. In a specific example, the detectable moiety is a fluorescently labeled bead, such as a microsphere, that has a specific fluorescence profile so that is capable of being used in a Luminex® xMAP assay. Typically a large number of the same probe will be attached to a single bead. The present invention also provides a plurality of probes having different combinations of probe regions attached to different sets of beads, for example fluorescently labeled beads. Where multiple probes and sets of beads are provided, each bead or set of beads can have a distinct fluorescent label that is associated with a particular probe such that no two probes are associated with the same detection molecule. Typically, the different beads or bead sets have different fluorescence color ratios and each different fluorescence color ratio is specific for one of the one of the plurality of the oligonucleotide probes. The present disclosure can also involve preparing or providing such probe/bead (bead set) combinations.

The present nucleic acid probe can also be attached to a solid particle or support. When the nucleic acid probe is attached the solid support it can be either covalently bound to the solid support, directly or through a linker, or attached to the solid support via an intermediate entity, for example where a nucleic acid is attached to the solid support and this attached nucleic acid then binds part of the nucleic acid probe.

The present invention also provides arrays of the present probes that are contained within distinct, defined locations on a support. In some embodiments, the probes will be attached to the support in the defined location. The probes can also be contained within a well of the support. Each defined, distinct area of the array will typically have a plurality of the same probes. As used herein, the term well is used solely for convenience and is not intended to be limiting. For example, a well can include any structure that serves to hold the nucleic acid probes in the defined, distinct area on the solid support. Non-limiting example of wells include depressions, grooves, walled surrounding and the like. In some of the arrays, the probes at different locations can have the same probing regions or consist of the same molecule. This embodiment is useful when testing whether nucleic acids from variety of sources contain the same target sequences. The arrays can also have probes with one or different probe regions at different locations within the array. This embodiment can be useful where nucleic acids from a single source are assayed for a variety of target sequences. Combinations of these array configurations are also provided where some of the probes in the defined locations contain the same probe regions whereas other locations contain probes with probe regions that are specific for different targets. Any suitable support can be used for the present arrays, such as glass or plastic, either of which can be treated or untreated to help bind, or prevent adhesion of, the probe. In some embodiments, the support will be a multi-well plate that allows the probes not to be bound to the support and be free in solution. Such arrays can be used for automated or high volume assays for target nucleic acid sequences: Suitable array configurations and useful supports that can be used in the present invention are discussed in U.S. Patent No. 6,544,790.

As used herein, region indicates a portion of the nucleic acid probe having a particular property. The probe regions can be made up of, or contain, nucleotides or nucleic acid sequences. Reference to different sequences refers to nucleotide or nucleic acid sequences. Although the present probes generally utilize the five standard nucleotides (A, C, G, T and U) in the nucleotide sequences, the identity of the nucleotides or nucleic acids used in the present invention are not so limited. Non-standard nucleotides and nucleotide analogs, such as peptide nucleic acids and locked nucleic acids can be used in the present invention, as desired. Several nucleotide analogs are known in the art (e.g., see, in Rawls, C & E News Jun. 2, 1997 page 35; in Brown, Molecular Biology LabFax, BIOS Scientific Publishers Limited; Information Press Ltd, Oxford, UK, 1991). In addition, the bases in a sequence may be joined by a linkage other than a phosphodiester bond, such as in a peptide nucleic acid, so long as the bond does not interfere with hybridization. These nucleotide analogs include any of the known base analogs of DNA and RNA such as, but not limited to, 4-acetylcytosine, 8-hydroxy-N6-methyladenosine, aziridinylcytosine, pseudoisocytosine, 5-(carboxyhydroxylmethyl) uracil, 5-fluorouracil, 5-bromouracil, 5-carboxymethylaminomethyl-2-thiouracil, 5-carboxymethylaminomethyluracil, dihydrouracil, hypoxanthine, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, 2-thiocytosine, 5-methyl thiouracil, 2-thiouracil, 4-thiouracil, 5-methyluracil, N-uracil-5-oxyacetic acid methylester, uracil-5-oxyacetic acid, 2-thiocytosine, orotic acid, 2,6-diaminopurine and the AEGIS™ bases isoC and isoG. AEGIS bases can be particularly useful in the self-complementary sequences of the present probes that are not included in the probe regions because they are not likely to interfere with binding between the probe(s) and target sequence(s). As such, the probes can contain DNA, RNA, analogs thereof or mixtures (chimeras) of these components.

Universal nucleotides can also be used in the present probes in the probe, self-annealing, spacer and/or arm regions or sequences although they are preferably not used in the spacer region to prevent the spacer region from having self-complementary sequence. As used herein, universal nucleotide, base, nucleoside or the like, refers to a molecule that can bind to two or more, i.e., 3, 4, or all 5, naturally occurring bases in a relatively indiscriminate or non-preferential manner. In some embodiments, the universal base, such as 2'-deoxyinosine (inosine), can bind to all of the naturally occurring bases in this manner. For example, the universal base can bind all of the naturally occurring bases with equal affinity, such as 3-nitropyrrole 2'-deoxynucleoside (3-nitropyrrole) and those disclosed in U.S. Patent Nos. 5,438,131 and 5,681,947. Generally, when the base is "universal" for only a subset of the natural bases, that subset will generally either be purines (adenine or guanine) or pyrimidines (cytosine, thymine or uracil). Examples of a nucleotide that can be considered universal for purines is known as the "K" base (N6-methoxy-2,6-diaminopurine), as discussed in Bergstrom et al., Nucleic Acids Res. 25:1935 (1997) and a nucleotide that can be considered universal for pyrimidines is known as the "P" base (6H,8H-3,4-dihydropyrimido[4,5-c][1,2]oxazin-7-one), as discussed in Bergstrom et al., supra, and U.S. Patent No. 6,313,286. Other suitable universal nucleotides include 5-nitroindole (5-nitroindole 2'-deoxynucleoside), 4-nitroindole (4-nitroindole 2'-deoxynucleoside), 6-nitroindole (6-nitroindole 2'-deoxynucleoside) or 2'-deoxynebularine. A partial order of duplex stability between the universal nucleotides and the natural bases has been found as follows: 5-nitroindole > 4-nitroindole > 6-nitroindole > 3-nitropyrrole. Combinations of these universal bases can also be used as desired.

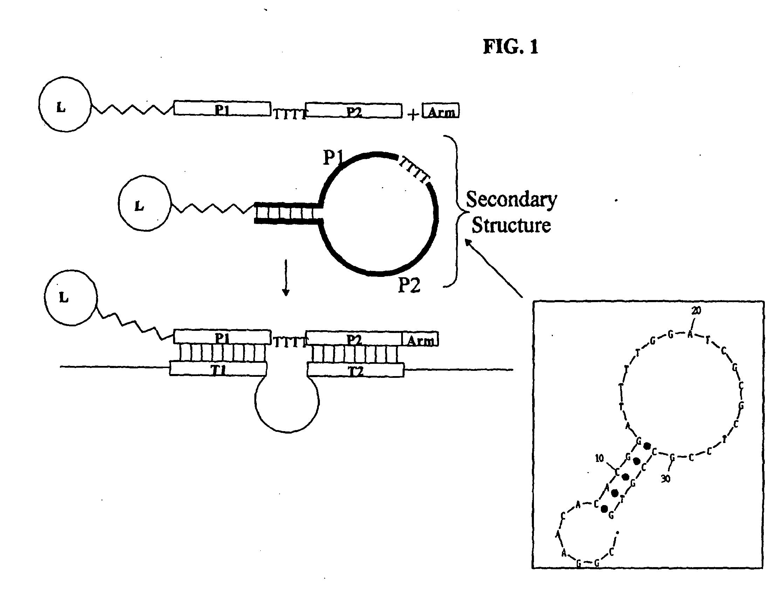

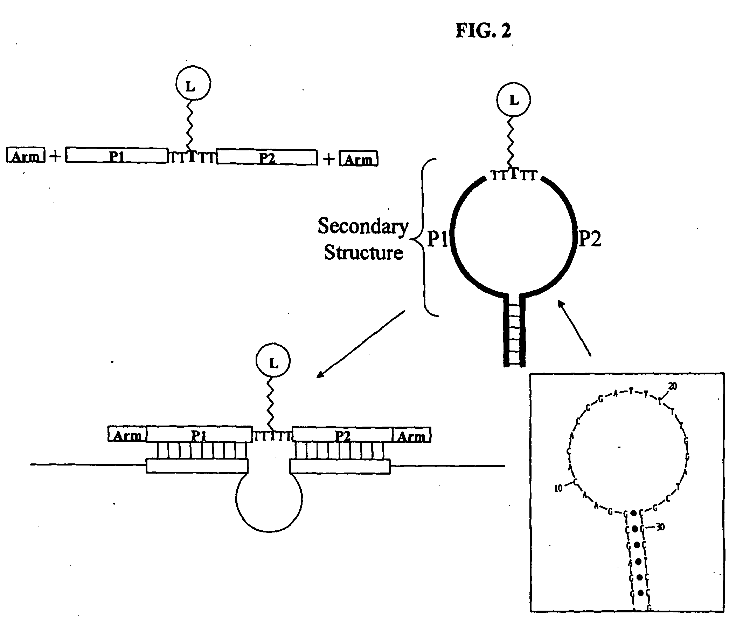

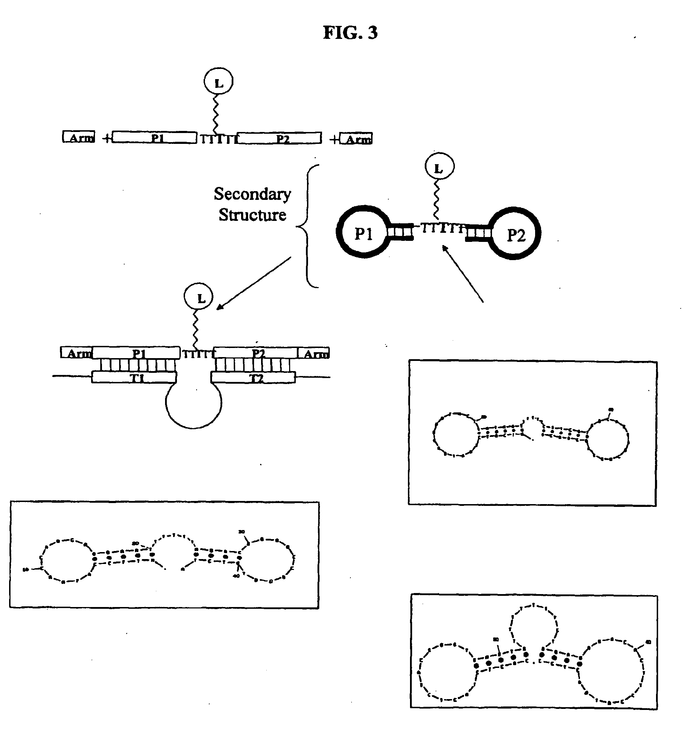

Several non-limiting configurations for the present probes are shown in the figures, where Arm indicates a self-complementary region outside any probe or spacer region, P1 indicates a first probe region, P2 indicates a second probe region, L indicates an optional label, such as a bead, T1 indicates the target sequence for the first probe region and T2 indicates the target sequence for the second probe region. Figures 1-3 depict the nucleic acid probes schematically in an unhybrized state, self-hybridized, and hybridized to the target sequences. Referring to FIG. 1, this probe configuration contains two probing regions separated by a spacer, depicted as four Thymine nucleotides, one arm sequence attached to the distal end of P2 that hybridizes with a portion of P1, and a label attached to the terminal end of P1 via a linker group. As can be seen from this FIG., when the probe is in solution that lacks target sequence(s) the probe self-hybridizes to form a stem-loop secondary structure. When a nucleic acid containing the target sequencc(s) is present, the probe opens up and P1 and P2 bind to T1 and T2, respectively. FIG. 2 depicts a probe that has P1 and P2 separated by a five T nucleotide spacer, two arm sequences on the termini of the probe that are self-complementary to one another and a label group attached to the middle T of the spacer via a linker group. Similarly to FIG. 1, when the probe is in solution that lacks target sequence(s) the probe self-hybridizes to form a stem-loop secondary structure. When a nucleic acid containing the target sequence(s) is present, the probe opens up and P1 and P2 bind to T1 and T2, respectively. FIG. 3 depicts another configuration that contains the same elements as shown in FIG. 2, e.g. P1 and P2 separated by a five T nucleotide spacer, two arm sequences on the termini of the probe that are self-complementary to one another and a label group attached to the middle T of the spacer via a linker group. However, in this probe configuration, the arm groups are complementary to sequences within P1 and P2 so that there are four self-complementary sequences. In the absence of target sequence(s), this probe forms two stem-loop structures joined at the stem portion by the spacer. In the embodiment depicted in FIG. 3, it is possible that the two stem regions can be opened independently of one another. In this embodiment, the label can be removed from the spacer region and two fluorophores, which can the same or different, and one, two or more quencher moieties can be attached to the probe such that they are brought together in close proximity when the probe is self-hybridized. Accordingly, this single probe can act as two molecular beacons and signal the binding of either target sequence alone or both together. Generally, when the probe acts as a dual molecular beacon then the fluorophores are different so that each fluorophore specifically and independently identifies the hybridization of one known probe region to its target sequence. It is contemplated that one quencher moiety can quench two fluorophore simultaneously, reducing the complexity of the molecule.

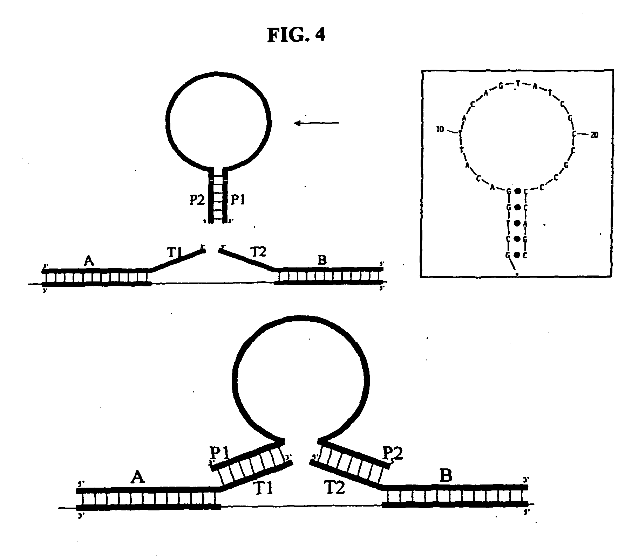

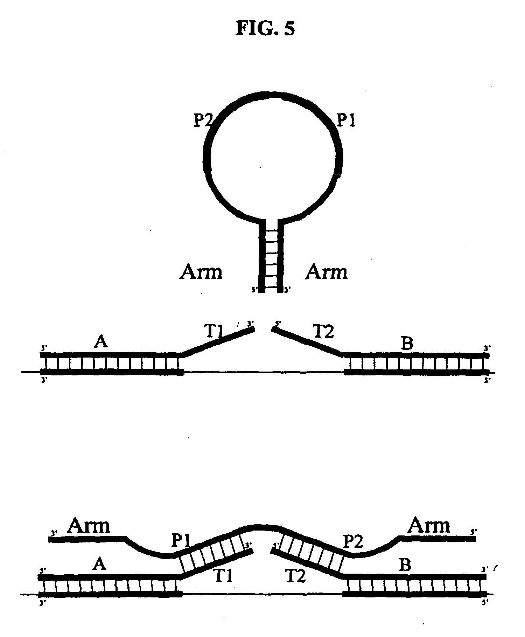

Figures 4 and 5 depict probe configurations that are unlabelled and that probe for target sequences on different nucleic acid molecules. In FIG. 4, P1 and P2 contain the self-complementary sequences, whereas in FIG. 5, the probe contains two arm sequences that are complementary to each other. Although both of these probe configurations provide stem-loop secondary structures in the absence of target sequence(s), in FIG. 4, the probe regions are located in the stem portion whereas in FIG. 5 the probe regions are in the loop portion. As shown in FIG. 5, there can also be a spacer between the probe regions and the arm regions. Additional probe configurations and sequences are shown in the Examples. Some of the probes shown in the figures also depict self-complementary sequences that span two different probe regions and sequences.

In the methods for assaying for the presence of one or more target sequences on one or more target nucleic acids using the probes described herein, these methods may involve contacting the one or more of the nucleic acid probes with a sample suspected of containing the one or more target nucleic acids and detecting whether any of the probe regions bind their respective target sequence. The probe(s) can be in solution, in an array or bound to solid particle or support as discussed above. Additional methods can also involve obtaining a nucleic acid sample from a subject suspected of having a target nucleic acid and/or amplifying the nucleic acids in the sample suspected of containing the one or more target nucleic acids prior to being contacted with the probe(s). The present methods can use the probes described herein with any other known or conventional nucleic acid probes as desired. In some of the present methods, the probes can be used as primers for nucleic acid extension, such as single-base extension, or amplification reactions. Although nucleic acid amplification of nucleic acids is often used to provide a larger sample, amplification is not required and the probes of the present invention can be hybridized to genomic nucleic acids directly.

In particular methods, the present probes are used to discriminate between nucleic acid sequences differences that differ by only a few positions, such as 1, 2, 3, 4 or 5. In these and other methods, the present probes can also help to resolve cis/trans ambiguities, particularly when the methods involve HLA typing or the target sequences are HLA sequences.

Without limiting the scope of the present invention, the present probes have been designed so that when they are in an appropriate solution in the absence of target nucleic acid sequence(s) the self-hybridizing sequences of the present probes will anneal or hybridize with one another so that the probes take on a "closed configuration." Examples of such closed configurations include various combinations of stems, formed by the self-hybridizing sequences, and loops, formed by the non-hybridized sequences between the hybridized stems. In some embodiments, the loops can be from about 5-25, such as 5, 10, 15, 20 or 25 nucleotides in length. In the presence of the target nucleic acid sequence(s), hybridization between the probe region(s) and target sequence(s) competes with the self-hybridizing sequences. This competitive reaction is believed to help probe specificity and is particularly useful for single-base difference discrimination in nucleic acids. The probes are designed such that the hybridization of probe region(s) and target sequence(s) is favored so that when the probe region and the target sequence are present together the probes undergo a conformational transition to an "open" state in which one or more of the probe regions binds one or more of the target sequences. In some embodiments, the probes can be designed to "open" when only one target sequence is present. In other embodiments, the probes will be designed to "open" only when more than one or all of the target sequences are present with the probe. In some embodiments, the transition to this open state results in the production of a fluorescent signal, such as where a fluorophore and quencher are no longer together in close proximity to one another. Empirically, the present probes have been found to be most efficient when the secondary structure of the self-hybridized probe at least partially protects the probe regions.

In some of the present probes one or more of the probing regions can be at least partially contained within a loop so that the probing regions are hidden or protected from non-specific hybridization with non-target sequences. In other embodiments at least part of the one or more of the probe regions will not be located in either the stem or the loop so that this probe region is more readily capable of binding to a target sequence. Once this probe region is bound to its target sequence, an increased concentration effect for the other probe region(s) can be observed which facilitates the binding of the other probe region(s) to their respective target sequences. This embodiment can be particularly useful where a relatively short target sequence or a sequence that can be difficult to detect, is located near a well-defined or conserved, generally longer, nucleic acid sequence.

Some of the present methods involve contacting the sample with the one or more nucleic acid probes attached to specifically labeled fluorescent beads as described above. A reporter molecule that detects probe-target hybridization is also added to the reaction. The reporter molecule can measure the magnitude of binding in the assay. The reporter molecule can signal the extent of the reaction by attaching to the bound probe-target molecules on the microspheres. In some embodiments, the reporter molecule works by signal with a color, e.g. a fluorescent wavelength, such that there are two sources of color for the reaction. One color-code for the bead or microsphere, which identifies the attached probe, and the reporter color which measures probe-target binding. The reporter molecule can be on the target sequence, attach to the target sequence or can attach to the probe-target hybrid. In some embodiments, the reporter molecule can be a labeled antibody.

In short, in some embodiments the color-coded microspheres bound to the probes, reporter molecules, and sample are combined. This mixture is then injected into an instrument that aligns the microspheres, typically through microfluidics, in single file where illumination sources, such as lasers, illuminate the colors inside and on the surface of each microsphere. The color signatures of the different microspheres are detected and can be translated into identification signals that provide real-time, quantitative data for each reaction. According to this aspect, a sample can be tested with up to 100 or more different probes simultaneously. Examples of such microspheres, instruments and techniques that can be used with the present probes are described in U.S. Patent Nos. 6,524,793; 6,514,295; 6,449,562; 6,411,904; 6,366,354; 6,268,222; 6,139,800; 6,057,107; 6,046,807; 5,981,180; and 5,736,330 and are also provided by Luminex Corporation (Austin, Texas). In an alternative embodiment, a nucleic acid suspected of having one or more target sequences can be attached to the luminescent bead and the probe can be free in solution. This embodiment can be easily achieved where the nucleic acid suspected of having one or more target sequences is obtained by amplification.

The nucleic acids targeted by the present probes are not particularly limited, and can be obtained form any source, either natural or synthetic. In some embodiments, the target sequences are polymorphic nucleic acid sequences, including polymorphic alleles. As above, the target nucleic acid sequences can be DNA, RNA, analogs thereof and combinations or chimeras of these. Accordingly, all possible probe-target configurations are contemplated by the present invention including DNA-DNA, DNA-RNA, RNA-DNA, RNA-RNA, Chimera-DNA, Chimera-RNA, DNA-Chimera, RNA-Chimera, Chimera-Chimera, etc. In some embodiments, the target nucleic acids can be obtained from an individual and are preferably alleles of interest in the individual's genome. Thus, the present methods can be used to provide the tissue type or genotype of the individual when individual is homozygous or heterozygous for the alleles of interest. The present methods can also be used to simultaneously detect alleles from different individuals, such as prospective tissue donors/recipients or in paternity testing. In some embodiments, target sequences can be pools of two or more alleles, and in some instances can be 10, 20, 50, 100, 200 alleles or more. Accordingly, preferred methods focus on identifying HLA alleles. The alleles of the HLA loci are classified as Class I - HLA-A, HLA-B, HLA-C, HLA-E, HLA-F and HLA-G, or Class II - HLA-DRA, HLA-DRB1, HLA-DRB2-9, HLA-DQA 1, HLA-DQB1, HLA-DPA1, HLA-DPB 1, HLA-DMA, HLA-DMB, HLA-DOA and HLA-DOB. There are over a hundred identified alleles that fall into some of these loci and these alleles are closely related and can differ in sequence by only one, or a few, positions. The HLA gene is discussed by Schreuder et al. in Tissue Antigens, 58:109 (2001) and the references disclosed therein. Additional information regarding HLA alleles, and in particular sequence information is available at www.ebi.ac.uk/imgt/hla and www.anthonynolan.org.uk/research.html.

Other preferred target sequences include those that encode Killer-cell Immurioglobulin-like Receptors (KIRs) and T-cell receptors (TCRs). KIRs have been shown to be highly polymorphic at the allelic and haplotypic level. KIRs are members of the immunoglobulin superfamily (IgSF) formerly called Killer-cell Inhibitory Receptors. They are composed of two or three Ig-domains, a transmembrane region and cytoplasmic tail which can in turn be short (activatory) or long (inhibitory). The Leukocyte Receptor Complex (LRC) which encodes KIR genes has been shown to be polymorphic, polygenic and complex like the MHC. KIRs and genes are discussed in Marsh et al., Tissue Antigens 62 (1), pp. 79-86, July 2003; Gagne et al., Hum Immunol 63 (4), pp. 271-280, April 2002; Uhrberg et al., Immunogenetics 54 (4), pp. 221-229, July 2002; Bradshaw et al., Tissue Antigens 61 (2), pp. 118-135, February 2003; Koguri et al., Mediators Inflamm 12 (2), pp. 117-121, April 2003; Cook et al., Eur J Immunogenet 30 (3), pp. 213-221, June 2003; Yawata et al., Crit Rev Immunol 22 (5-6), pp. 463-482, 2002; http://www.ncbi.nlm.nih.gov/mhc/MHC.cgi?cmd=aligndisplay&user_id=0&probe_id= 0&source_id=0&locus_id=36&kit_id=0&banner=1, and http://www.ebi.ac.uk/ipd/kir/.

Depicted below is combination of motifs, shown generically as A and B, in different alleles.

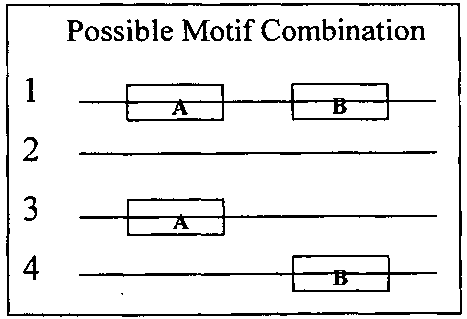

In some embodiments of the present invention, the probes recognize combination 1 (AB) but not the others. Throughout the specification and the figures, A and B are also referred to as T1 and T2. Combination 2(--) generally and/or theoretically will not be a problem because the probe will not bind. However, 3(A) and 4(B) can bind partially the probe for AB.

In other embodiments, the probe can discriminate between samples with different combination of alleles when the motif AB is present versus other alleles without AB. The possible allele combination are represented below. Also shown are the expected results of using the probes of the current invention with the possible allele combinations.

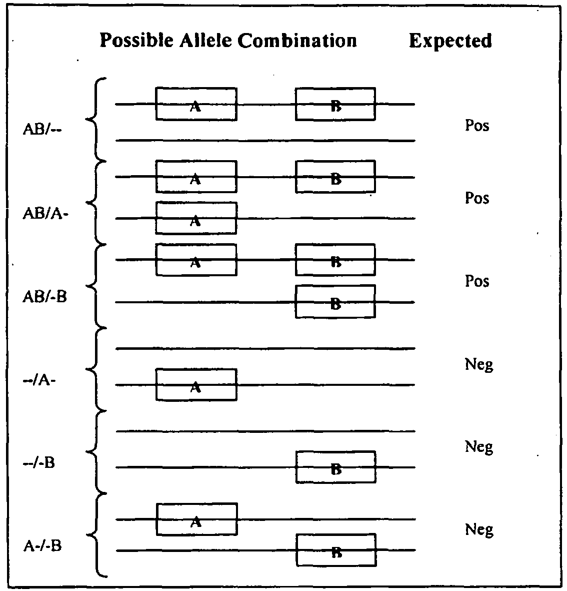
Possible Allele Combination Expected

AB/-- A B Pos

AB/A- A B A Pos

AB/-B A B B Pos

--/A- A Neg

--/-B B Neg

A-/-B A B Neg

By using a suitable probe or combination of probes, the present invention is capable of properly distinguishing all of these various combinations. In some of the present probes, the probe regions are restricted to a non-linear configuration in order prevent non-specific hybridization of probe regions with partial sets of target sequences, which can occur at sub-optimal conditions (e.g. lower temperature, higher salt concentration). Preventing non-specific hybridization minimizes signal noise. At a specified condition, for example when one or both target sequences are present, the self-complementary binding of the probe is disrupted and the probe regions hybridize to available target sequences. Consequently, in some embodiments, hybridization with target sequences on the same molecule results in higher Tm and therefore provides the highest signal level. Hybridization with target sequences on different molecules results in lower Tm and thus provides a lower signal level. Hybridization with only one target sequence further lowers Tm and also signal level. Lack of hybridization with a target sequence results in "zero" signal level. Accordingly, some of the present probes can distinguish all possible binding combinations of the present probes and their target sequences.

The present invention also provides kits for carrying out the methods described herein. In one embodiment, the kit is made up of one or more of the described probes with instructions for carrying out any of the methods described herein. The instructions can be provided in any intelligible form through a tangible medium, such as printed on paper, computer readable media, or the like. The present kits can also include one or more reagents, buffers, hybridization media, nucleic acids, primers, nucleotides, labels, molecular weight markers, enzymes, solid supports, databases, computer programs and/or disposable lab equipment, such as multi-well plates, in order to readily facilitate implementation of the present methods. Solid supports can include beads and the like whereas molecular weight markers can include conjugatable markers, for example biotin and streptavidin or the like. Examples of preferred kit components can be found in the description above and in the following examples.

Whether bound to a support or in solution, the methods, kits and compositions of this disclosure are particularly useful for the rapid, sensitive, reliable and versatile detection of-target sequences that are particular to organisms, such as pathogens, bacteria, viruses, fungi or the like, which might be found in food, beverages, water, pharmaceutical products, personal care products, dairy products, environmental samples, clinical environments or the like. Consequently, the methods, kits and compositions of this disclosure will be particularly useful for the analysis of raw materials, equipment, products or processes used to manufacture or store food, beverages, water, pharmaceutical products, personal care products, dairy products or environmental samples. As such, the methods, kits and compositions of this disclosure will also be particularly useful for the analysis of clinical specimens or equipment, fixtures or products used to treat humans or animals. For example, the assay may be used to detect a target sequence which is specific for a genetically based disease or is specifc for a predisposition to a genetically based disease. Non-limiting examples of disease include, beta-thalassemia, sickle cell anemia, Factor-V Leiden, cystic fibrosis and cancer related targets such as p53, p10, BRC-1 and BRC-2.

In still another embodiment, the target sequence may be related to a chromosomal DNA, wherein the detection, identification or quantitation of the target sequence can be used in relation to forensic techniques such as prenatal screening, paternity testing, identity confirmation or crime investigation.

This invention is further illustrated by the following non-limiting examples.

### EXAMPLES

### Example 1

In this Example, the following probe was used to detect the presence or absence of the HLA-A locus in a variety of test samples:

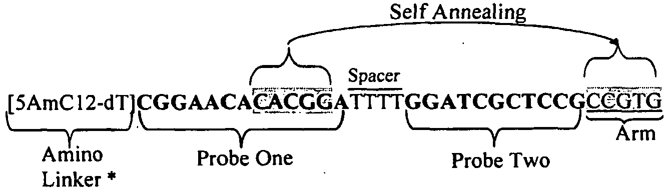

The amino linker for the probe in this example was:

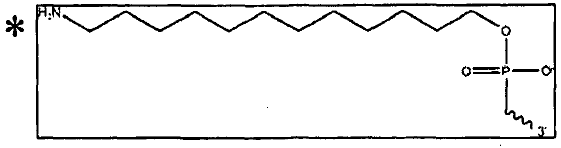

In the probe used in this example, an amino linker is placed 5' to (upstream of) a first probe region (Probe One). The first probe region is linked with a second probe region (Probe Two) via a 5'-TTTT-3' linker. Downstream of the 3' end of the second probe region is added a shared stem (arm) complementary to 5 nucleotides within the first probe region. At room temperature this probe self-hybridizes to form a loop, restricting exposure of half of the first probe sequence and the entire second probe sequence.

Material and methods. The following materials and methods were used in the present examples.

Coupling of probes to beads. Probes were covalently coupled to carboxylate-modified polystyrene beads by using water-soluble carbodiimide (EDC). 200 µl (2.5 x 10⁶ beads total) from stock were centrifuged and resuspended in 25 µL 0.1 mol/L MES buffer (pH 4.5), then 1.0 µL of 0.2 nmole/µL [of what] and 2.5 µL of 10 g/L solution of EDC were added, the solution was vortexed and incubated for 30 minutes at room temperature in the dark, shaking in an Eppendorf Thermomixer at 700 rpm. The same amount of EDC was added again and the solution was mixed for another 30 min. 1.0 ml of Tween20 (0.02% v/v) was added, the solution was vortexed and then the beads were centrifuged at ~10,000 rpm, then 1.0 ml of 0.1% SDS was added, the solution was again vortexed and centrifuged at ~10,000 rpm. Following the centrifugation, the beads were resuspended in 1X TE (pH 8.0) using half of the original bead volume.

DNA samples. DNA was extracted by conventional techniques from samples obtained from the International Histocompatibility Workshops (IHW) cell lines panel, UCLA interchange panel and samples locally available.

PCR amplification. PCR amplification was performed using 10 pmol of each locus (125 ng DNA), 20 mM (NN₄)₂SO₄, 84 mM Tris Base, 0.0125 % Tween 20, 0.25 mM dNTPs, 2.75 mM MgCl₂ and 1 units Taq Polymerase in a total volume of 25 µl. Thermocycle conditions were: 95°C 2 min; 35 cycles of 95°C for 20 sec, 64°C for 20 sec and 72°C for 45 sec, and extension at 72°C for 5 min.

Hybridization reaction. 5 µl of PCR amplicon was mixed with 15 µl of bead mix (approximately 2 µl of TE stock bead mix at a concentration of 2000 beads/ID/µl in 13 µl of hybridization buffer (60mM Sodium Citrate, 0.6M NaCl)). Hybridization was performed in a thermocycler as follows: 97°C for 10 min, 50°C for 20 min, 60°C for 10 min. When the hybridization was complete, 170 µl of labeling solution (SAPE) was added to each sample at 60°C in the thermocycler. Samples were diluted, removed from thermocycler, and incubated at room temperature for 5 minutes while being protected from light. The samples were centrifuged for 30 seconds to remove supernatant. About 50 µl sample remained. 70 µl of wash buffer (22.5mM Sodium Citrate, 0.225M NaCl) was added and the samples were mixed gently by pipetting, after which they were transferred to a plate for reading by a Luminex system.

A positive result (a mean fluorescent intensity (MFI) above background) for the probe is expected when the motif AB is present, whereas a negative result is expected when the motif combination only contains A-, B-, or --. The point at which a result will be labeled positive is determined experimentally. Generally, a result is deemed positive when the signal noise ratio is greater than two. However, the skilled artisan will understand that probes producing signal noise ratios between one and two may still be useful in the methods of the present invention. Furthermore, positive results in individual experiments may encompass signal noise ratios greater than three.

The results for this example are shown in the following table.

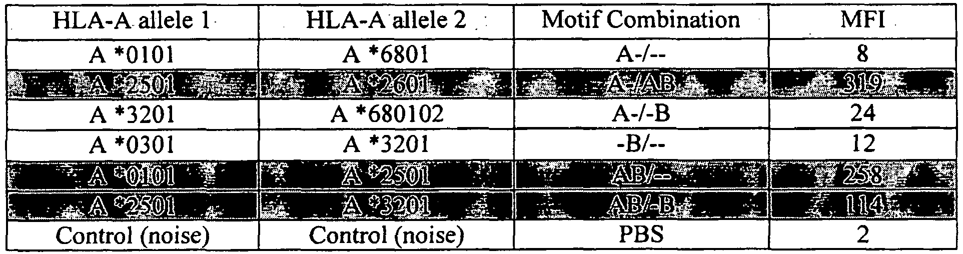

As shown by the table, actual hybridization results as measured by the MFI tracked the expected results. In all of the examples, the dark areas of the chart correspond to positive hybridization.

Restricting probe regions in a non-linear configuration prevents non-specific hybridization of probe region with partial sets of target sequences at sub-optimal conditions (e.g. lower temperature, higher salt concentration) thereby minimizing signal noise. At a specified condition, probe regions open up and hybridize to available target sequences. Consequently, hybridization with A and B on the same molecule result in higher Tm and therefore the highest signal level. Hybridization with A and B on different molecules typically results in lower Tm and a lower signal level. Hybridization with only one of A or B on different molecules further lowers Tm and thus the signal. Hybridization with neither A nor B results in "zero" signal level. Accordingly, the probe design of this example can distinguish all different binding configurations based on the strength of the signal as is shown in the above table.

In the present example, the probes of the present invention can discriminate between the samples that contain both target sequences in the same allele from other target sequence configurations. In this example, the signal to noise ratio ranged from about 6 to 20.

### Example 2

In this Example, the following probes were used to detect the presence or absence of two HLA-A and HLA-DRB1 loci in a variety of test samples: 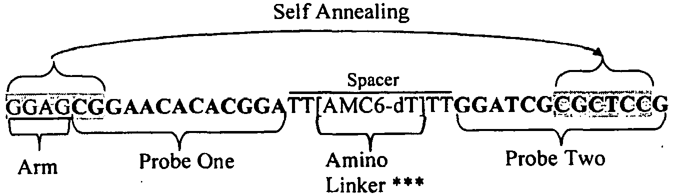 and

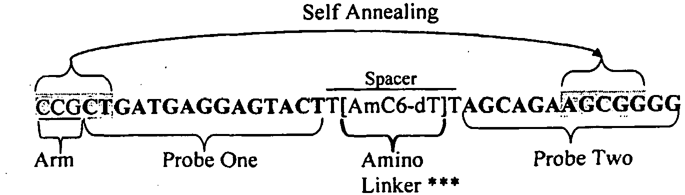

The linker for the probes in this example was:

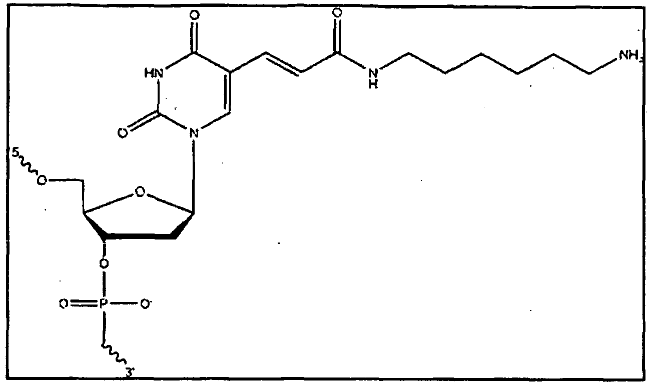

The probe used in this Example is similar to the one shown in FIG. 2. In this example, an amino linker, linked to a thymine nucleotide, is placed in the middle of the spacer. The spacers for the different probes were: 5' TTTTT 3' or b. 5' TTT 3'. This modification allows one to put the arm upstream of the first probe region, which is complementary to the 3' half of second probe region, thereby preventing exposure of the entire first probe region and second probe region in the absence of target sequence. The results for this Example are shown in the following tables.

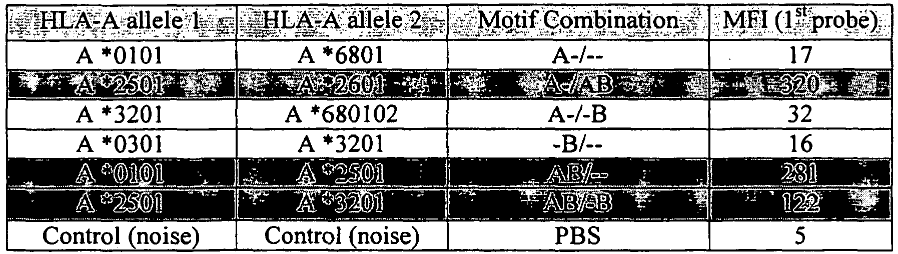

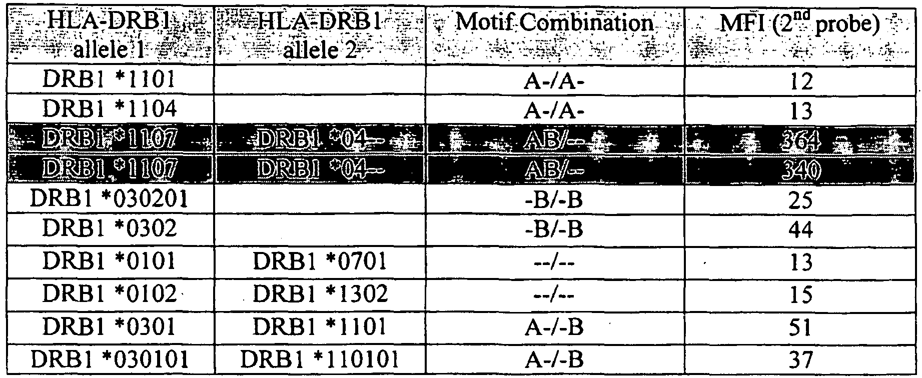

### Example 3

In this Example, the following probes were used to assay for the presence of the HLA-A and HLA-DRB1 loci in a variety of test samples: 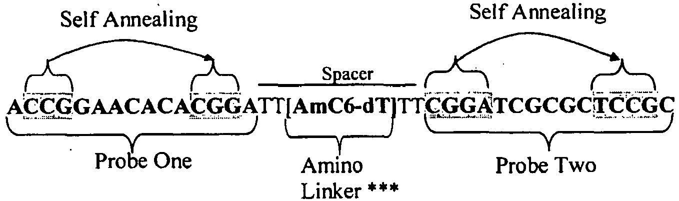 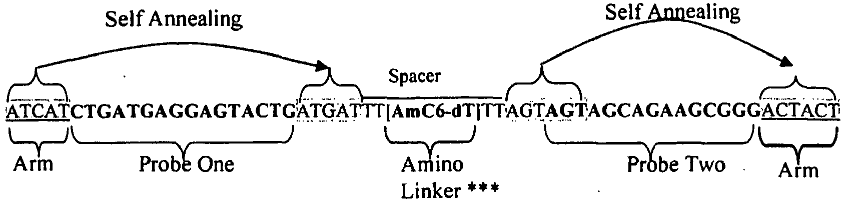 and 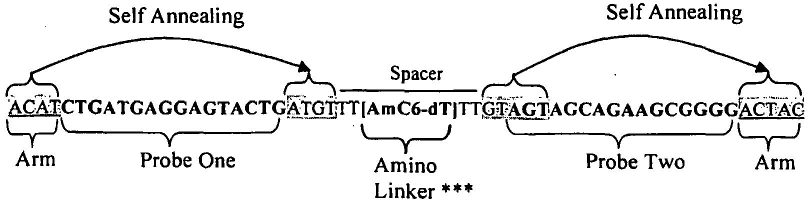 and 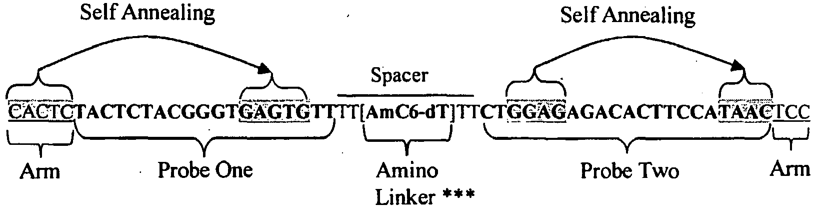 and 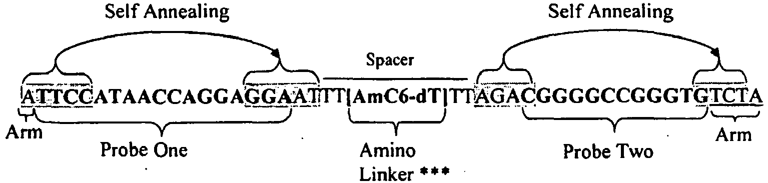 where the linker(***) was

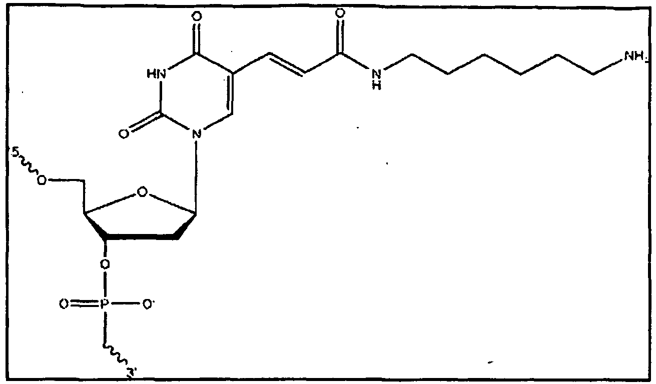

In this example the linker is at the same position as that of Example 2, with 5' TTTTT 3' as a spacer and the sequences of the first probe region and second probe region hybridize to themselves to form a loop making an additional arm unnecessary. The results for this experiment are shown below.

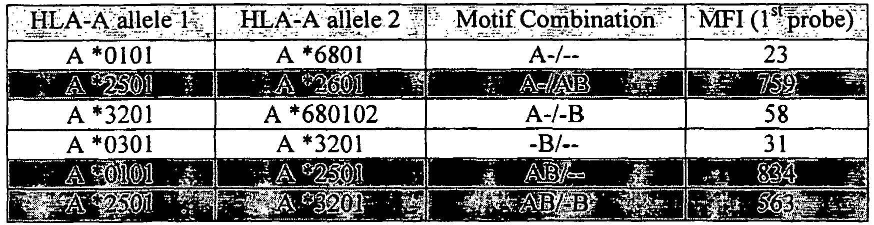

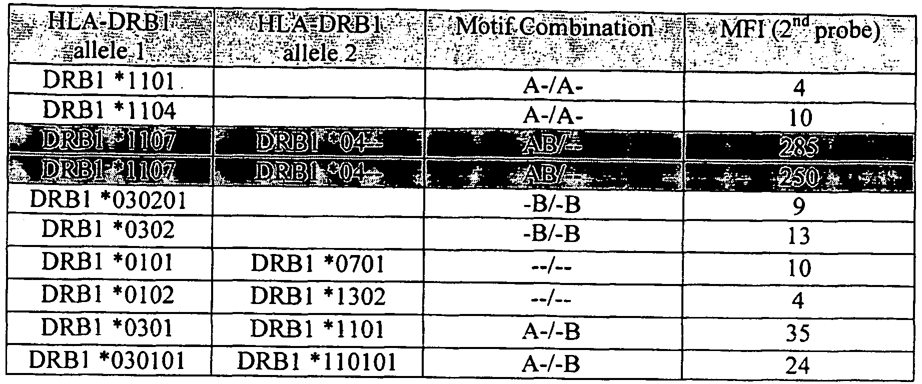

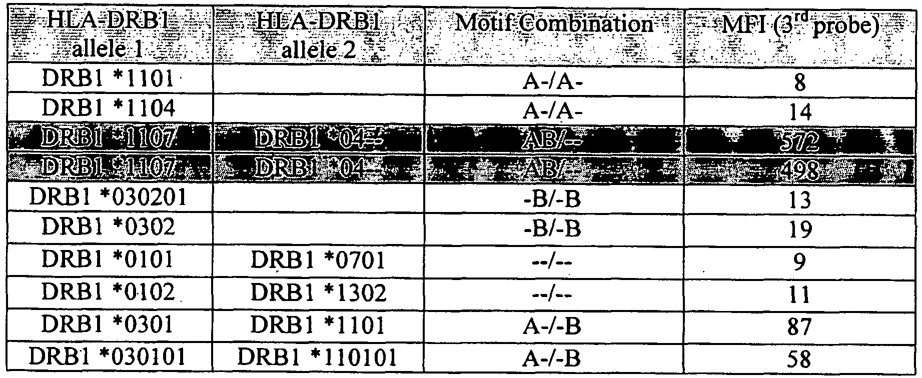

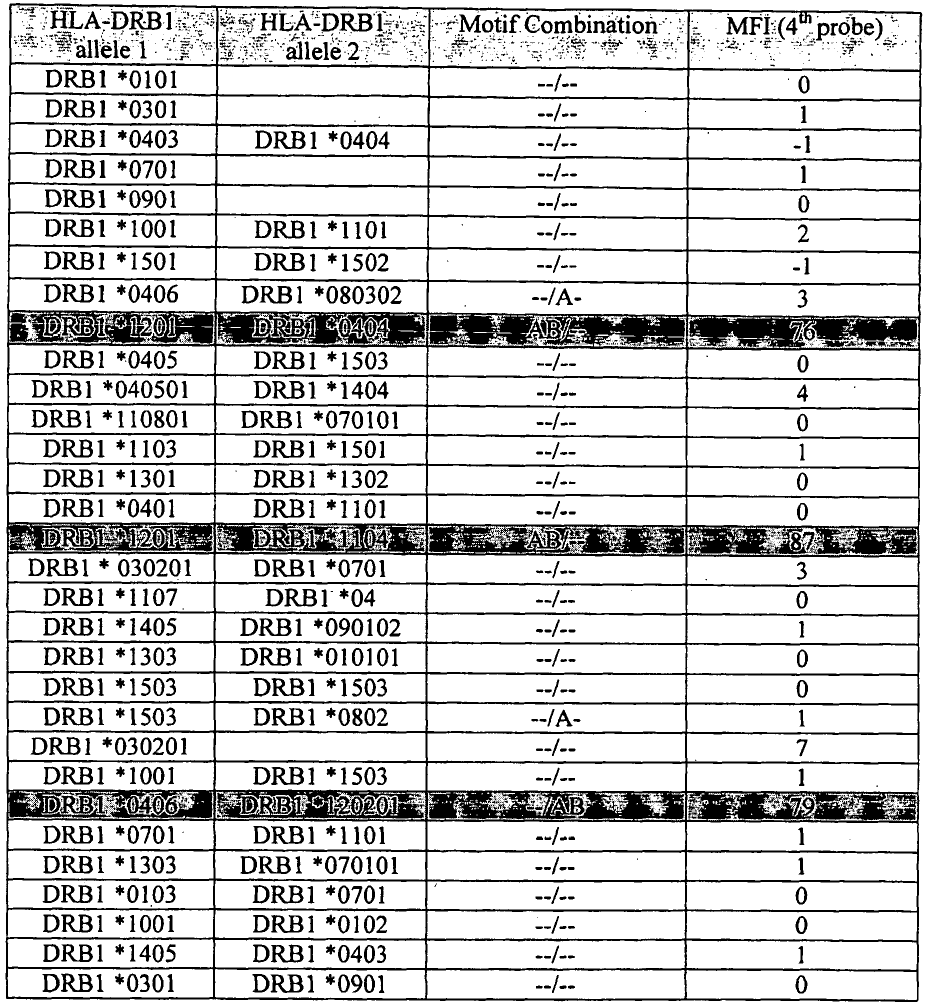

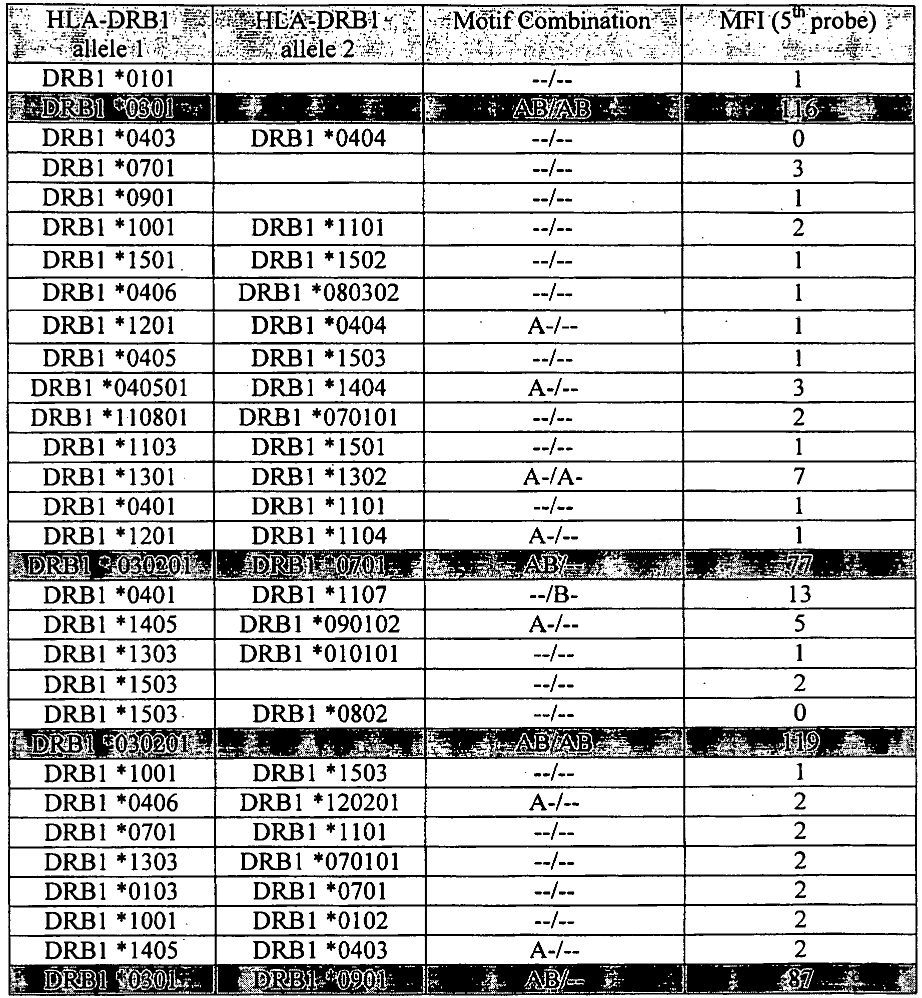

The probe design used in this Example is similar to the one depicted in FIG. 3. In the second and third probes in this example, the probe sequences were the same. The only difference was in the size of the arm. In the second probe, the arm was 6 nucleotides long and in the third probe, the arm was 5 nucleotides long. In this example, the third probe configuration provided the highest mean fluorescence intensity of the tested probe configurations and results in a higher signal to noise ratio. This demonstrates that the MFI is at least partially dependent upon the length of the arm.

### Example 4

In this Example, the following probes were used to detect the presence or absence of the DRB1 locus in a variety of test samples: 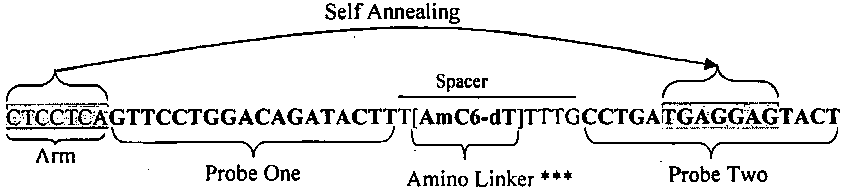 and 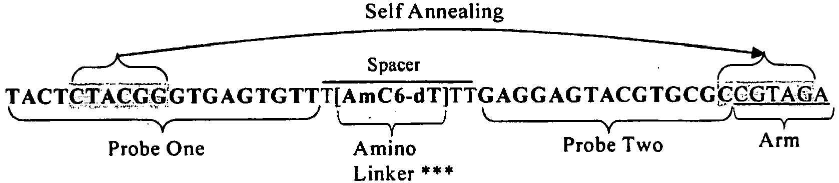 where the linker(***) was

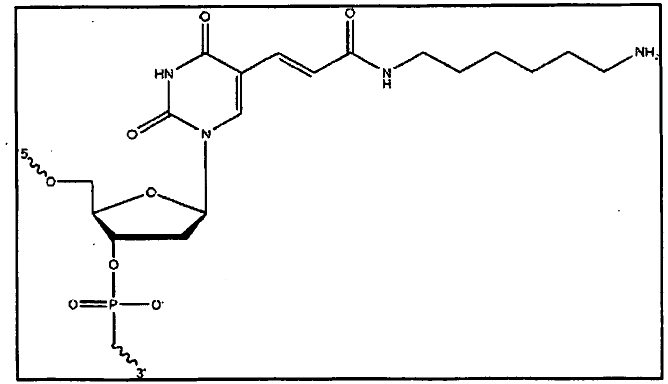

In this example the linker is at the same position as that of Example 2 and Example 3, with either 5' TTTTG 3' or 5' TTTTT 3' as a spacer and the sequences of the first probe region and second probe region hybridize to themselves to form a loop. As demonstrated by the first probe of this example, no requirement exists that the spacer only comprise thymine nucleotides. The probes in Example 4 generally depict the design of the probe in FIG. 2. The results for the Example 4 probes are shown below.

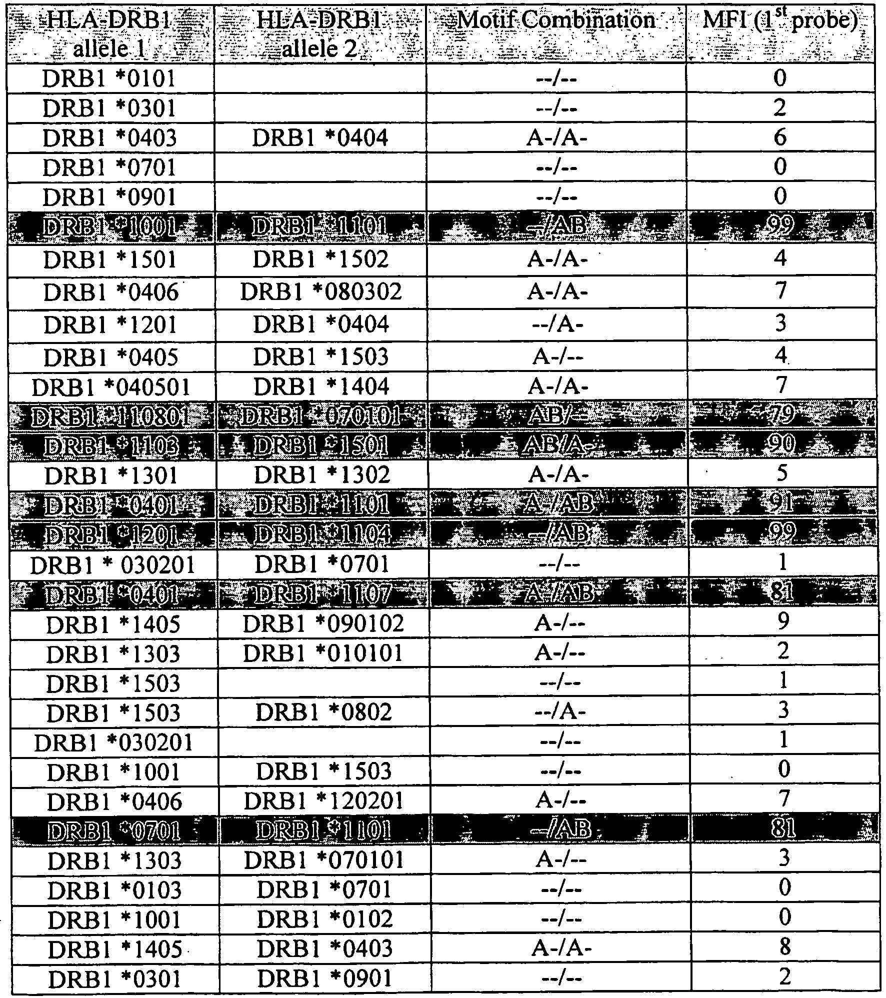

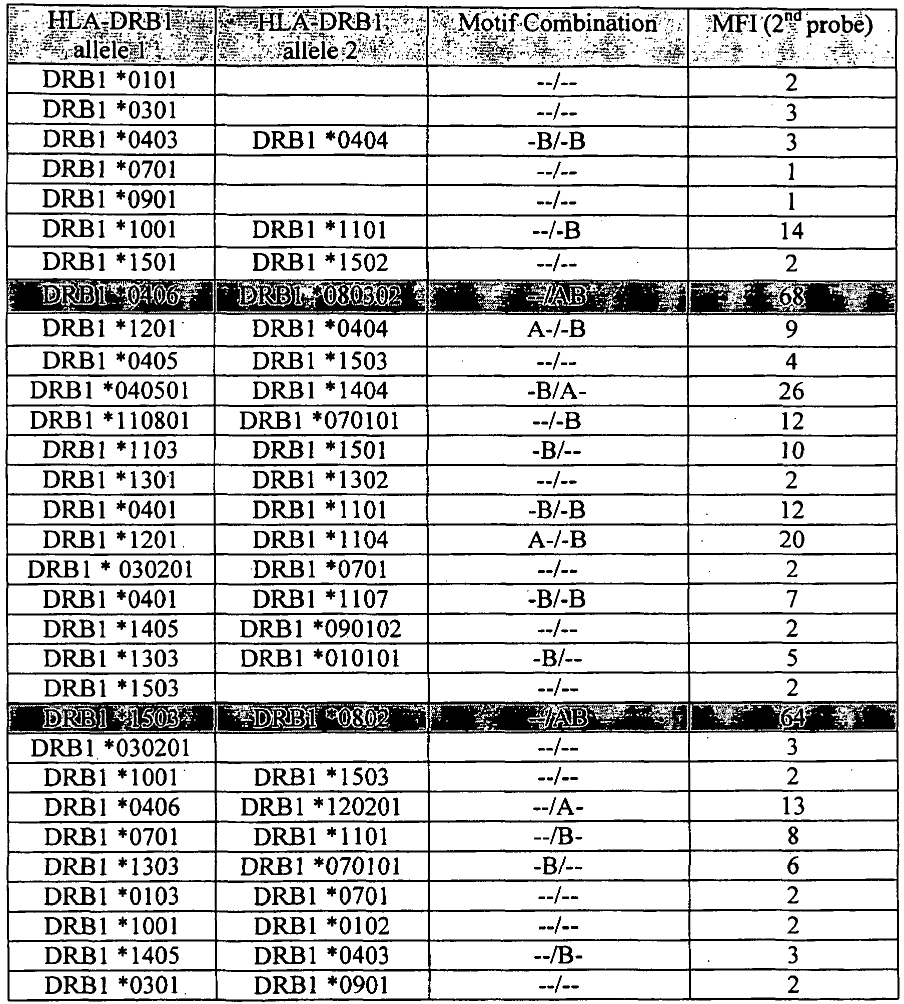

The present probes can have any or all of the components described herein. Likewise, the present methods can be carried out by performing any of the steps described herein, either alone or in various combinations. One skilled in the art will recognize that all embodiments of the present invention are capable of use with all other appropriate embodiments of the invention described herein. Additionally, one skilled in the art will realize that the present invention also encompasses variations of the present probes, configurations and methods that specifically exclude one or more of the components or steps described herein.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halVes, thirds, quarter, fifths, tenths, etc. As a non-limiting example, each range discussed herein can be readily broken down into a lower third, middle third and upper third, etc. As will also be understood by one skilled in the art all language such as "up to," "at least," "greater than," "less than," "more than" and the like include the number recited and refer to ranges which can be subsequently broken down into subranges as discussed above. In the same manner, all ratios disclosed herein also include all subratios falling within the broader ratio.

One skilled in the art will also readily recognize that where members are grouped together in a common manner, such as in a Markush group, the present disclosure encompasses not only the entire group listed as a whole, but each member of the group individually and all possible subgroups of the main group. Accordingly, for all purposes, the present disclosure encompasses not only the main group, but also the main group absent one or more of the group members. The present disclosure also envisages the explicit exclusion of one or more of any of the group members in the disclosure.

## Claims

1. A method for detecting more than one target sequence on a target nucleic acid comprising:
(a) contacting a nucleic acid probe comprising
(i) a first probe region specific for a first target sequence, and
(ii) a second probe region specific for a second target sequence, wherein the first probe region and the second probe region are non-contiguous, the first target sequence and the second target sequence are non-contiguous, or both the first probe region and the second probe region and the first target sequence and the second target sequence are non-contiguous, and wherein the first target sequence and the second target sequence are each an HLA allele; and
(iii) self-complementary sequences;
wherein in the presence of the target sequences, hybridisation between the first and second probe regions and the first and second target sequences competes with hybridisation of the self-complementary regions;
with a sample suspected of containing the target nucleic acid; and
(b) detecting whether one or more of the first probe region and the second probe region binds their respective target sequence, the method optionally comprising the further step of:
(c) amplifying the nucleic acids in the sample suspected of containing the target nucleic acid prior to (a).

2. A method as claimed in claim 1 wherein the first target sequence and the second target sequence are on the same target nucleic acid.

3. A method as claimed in claim 1 or claim 2 wherein the nucleic acid probe is capable of distinguishing a target nucleic acid which comprises both the first target sequence and the second target sequence from nucleic acids that do not comprise both the first target sequence and the second target sequence.

4. A method as claimed in claim 1 wherein the first target sequence and the second target sequence are on different target nucleic acids.

5. A method as claimed in any one of the preceding claims wherein the nucleic acid probe comprises two or four self-complementary sequences.

6. A method as claimed in any one of the preceding claims wherein at least one of the first probe region and the second probe region comprises at least part of one of the self-complementary sequences.

7. A method as claimed in any one of claims 1 to 5 wherein the first probe region comprises at least part of one of the self-complementary sequences and the second probe region comprises at least part of another of the self- complementary sequences.

8. A method as claimed in claim 7 wherein the self-complementary sequence of the first probe region is complementary to the self-complementary sequence of the second probe region.

9. A method as claimed in claim 7 wherein the self-complementary sequence of the first probe region is not complementary to the second probe region and the self-complementary sequence of the second probe region is not complementary to the first probe region.

10. A method as claimed in claim 5 wherein only one of the first probe region and second probe region comprises one of the self- complementary sequences.

11. A method as claimed in claim 5 wherein neither the first probe region nor the second probe region comprise at least part of the self-complementary sequences.

12. A method as claimed in claim 5 wherein none of the self-complementary sequences are located between the first probe region and the second probe region.

13. A method as claimed in any one of the preceding claims further comprising one or more of:
(e) a spacer moiety that separates the first probe region and the second probe region,
(d) one or more arm sequences that comprise one or more of the self-complementary sequences, wherein the one or more arm sequences do not comprise the probe regions,
(e) one or more detectable labels, wherein the one or more detectable labels can be the same or different.

14. A method as claimed in claim 13 wherein the probe comprises a spacer moiety that separates the first probe region and the second probe region and the spacer moiety comprises a nucleic acid.

15. A method as claimed in claim 13 or claim 14 wherein the probe comprises one or more arm sequences that comprise one or more of the self-complementary sequences, wherein the one or more arm sequences do not comprise the probe regions and the one or more self-complementary sequences of the one or more arm sequences are complementary with a self-complementary sequence located at least partially within one of the probe regions.

16. A method as claimed in claim 13 or claim 14 wherein the probe comprises one or more arm sequences that comprise one or more of the self-complementary sequences, wherein the one or more arm sequences do not comprise the probe regions and a first self-complementary sequence of a first of the one or more arm sequences is complementary with a second self-complementary sequence of a second of the one or more arm sequences.

17. A method as claimed in claim 13 or claim 14 wherein the probe comprises one or more arm sequences that comprise one or more of the self complementary sequences, wherein the one or more arm sequences do not comprise the probe regions and at least one of the one of more self-complementary sequences of the one or more arm sequences is complementary to at least a portion of the spacer moiety.

18. A method as claimed in any one of claims 13 to 17 wherein the probe comprises one or more detectable labels, wherein the one or more detectable labels can be the same or different and wherein the detectable label is covalently bound to a terminus of the nucleic acid probe.

19. A method as claimed in claim 18 or claim 19 wherein the probe comprises one or more detectable labels, wherein the one or more detectable labels can be the same or different and the detectable label is covalently bound to a spacer moiety of the nucleic acid probe.

20. A method as claimed in claim 19 wherein the probe comprises one or more detectable labels, wherein the one or more detectable labels can be the same or different and the detectable label comprises a linker group, wherein the linker group is covalently bound to the nucleic acid probe, and optionally wherein the linker group comprises an amino linker.

21. A method as claimed in any one of claims 13 to 20 wherein the detectable label is a fluorescent molecule, and preferably the nucleic acid probe does not comprise a fluorescent quencher moiety.

22. A method as claimed in any one of claims 13 to 21 wherein the detectable label is a bead, optionally a latex or polystyrene bead.

23. A method as claimed in any preceding claim wherein the nucleic acid probe is attached to a solid support.

24. A method as claimed in any preceding claim wherein the first probe region and second probe region have different sequences or wherein the first probe region and second probe region have the same sequence.

25. A method as claimed in any preceding claim wherein the target nucleic acid is an allele and the first target sequence and second target sequence are motifs on the same allele.

26. A method according to claim 1 wherein the nucleic acid probe comprises:
(a) a first probe region specific for a first target sequence; and
(b) a second probe region specific for a second target sequence, the first probe region and the second probe region being non-contiguous; and
(c) self-complementary sequences; wherein:
in the absence of one or both target sequence(s), the self-complementary sequences of the nucleic acid probe anneal so that the nucleic acid probe adopts a closed configuration, and in the presence of one or both target sequence(s) hybridisation of the nucleic acid probe with one or both target sequence(s) is favoured such that the probe adopts an open configuration.

27. A method according to claim 26 wherein one or both probe regions are at least partially contained within a loop when the nucleic acid probe is in a closed configuration to prevent hybridisation with non-target sequences.

28. A method according to claim 26 or claim 27 wherein the nucleic acid probe is capable of distinguishing between allele combinations AB/--, AB/A-. AB/-B, --/A, --/B, A-/-B wherein A and B denote different alleles.

29. A method according to any one of the preceding claims wherein one or both target sequences are class 1-HLA-A, HLA-B, HLA-C, HLA-E, HLA-F and HLA-G, or Class 11-HLA-DRA, HLA-DRB1, HLA-DRB2-9, HLA-DQA1, HLA-DOB1, HLA-DPA1, HLA-DPB1, HLA-DMA, HLA-DMB, HLA-DOA and HLA-DOB.

30. A nucleic acid probe as defined in any one of claims 26 to 29.

31. A kit comprising a nucleic acid probe of claim 30, in combination with instructions for detecting more than one target sequences on one or more target nucleic acid sequences using the nucleic acid probe, and optionally one or more reagents for detecting the one or more target sequences.

32. An array of nucleic acid probes comprising one or more of a plurality of nucleic acid probes of claim 30 located in defined distinct locations on a solid support.

33. The array of nucleic acid probes of claim 32 wherein one or more of the plurality of nucleic acid probes is attached to a solid support.

34. The array of nucleic acid probes of claim 32 or claim 33 wherein one or more of the plurality of nucleic acid probes is contained within a well of the solid support.

35. The array of nucleic acid probes of any of claims 32 to 34 wherein the nucleic acid probes in different defined, distinct locations on the solid support have one or more different probe regions.

36. The array of nucleic acid probes of any of claims 32 to 34 wherein the nucleic acid probes in different defined, distinct locations on the solid support have one or more of the same probe regions.

37. The array of nucleic acid probes of any one of claims 32 to 34 wherein one or more of the plurality of nucleic acid probes have the same probe regions.

## Patentansprüche

1. Verfahren zum Erfassen von mehr als einer Zielsequenz auf einer Zielnukleinsäure, umfassend:
(a) das Kontaktieren einer Nukleinsäuresonde, umfassend
(i) einen ersten Sondenbereich spezifisch für eine erste Zielsequenz, und
(ii) einen zweiten Sondenbereich spezifisch für eine zweite Zielsequenz, wobei der erste Sondenbereich und der zweite Sondenbereich nicht angrenzend sind, wobei die erste Zielsequenz und die zweite Zielsequenz nicht angrenzend sind oder sowohl der erste Sondenbereich als auch der zweite Sondenbereich und die erste Zielsequenz sowie die zweite Zielsequenz nicht angrenzend sind und wobei die erste Zielsequenz und die zweite Zielsequenz jeweils ein HLA-Allel sind; und
(iii) selbstkomplementäre Sequenzen;
wobei in Anwesenheit der Zielsequenzen die Hybridisierung zwischen den ersten und zweiten Sondenbereichen und den ersten und zweiten Zielsequenzen mit der Hybridisierung der selbstkomplementären Bereiche konkurriert;
mit einer Probe, die im Verdacht steht, die Zielnukleinsäure zu enthalten; und
(b) das Nachweisen, ob ein oder mehrere der ersten Sondenbereiche und der zweiten Sondenbereiche ihre entsprechende Zielsequenz binden, wobei das Verfahren optional den nachfolgenden weiteren Schritt umfasst:
(c) das Amplifizieren der Nukleinsäuren in der Probe, die im Verdacht steht, vor (a) die Zielnukleinsäure zu enthalten.

2. Verfahren nach Anspruch 1, wobei die erste Zielsequenz und die zweite Zielsequenz sich jeweils auf derselben Zielnukleinsäure befinden.

3. Verfahren nach Anspruch 1 oder Anspruch 2, wobei die Nukleinsäuresonde in der Lage ist, eine Zielnukleinsäure, die sowohl die erste Zielsequenz als auch die zweite Zielsequenz umfasst, von Nukleinsäuren zu unterscheiden, welche weder die erste Zielsequenz noch die zweite Zielsequenz umfassen.

4. Verfahren nach Anspruch 1, wobei die erste Zielsequenz und die zweite Zielsequenz sich jeweils auf verschiedenen Nukleinsäuren befinden.

5. Verfahren nach einem der vorstehenden Ansprüche, wobei die Nukleinsäuresonde zwei oder vier selbstkomplementäre Sequenzen umfasst.

6. Verfahren nach einem der vorstehenden Ansprüche, wobei wenigstens einer der ersten Sondenbereiche und der zweiten Sondenbereiche wenigstens eine der selbstkomplementären Sequenzen umfasst.

7. Verfahren nach einem der Ansprüche 1 bis 5, wobei der erste Sondenbereich wenigstens einen Teil einer der selbstkomplementären Sequenzen umfasst und der zweite Sondenbereich wenigstens einen Teil einer weiteren der selbstkomplementären Sequenzen umfasst.

8. Verfahren nach Anspruch 7, wobei die selbstkomplementäre Sequenz des ersten Sondenbereichs komplementär zur selbstkomplementären Sequenz des zweiten Sondenbereichs ist.

9. Verfahren nach Anspruch 7, wobei die selbstkomplementäre Sequenz des ersten Sondenbereichs nicht komplementär zum zweiten Sondenbereich ist und die selbstkomplementäre Sequenz des zweiten Sondenbereichs nicht komplementär zum ersten Sondenbereich ist.

10. Verfahren nach Anspruch 5, wobei nur einer der ersten Sondenbereiche und zweiten Sondenbereiche eine der selbstkomplementären Sequenzen umfasst.

11. Verfahren nach Anspruch 5, wobei weder der erste Sondenbereich noch der zweite Sondenbereich wenigstens einen Teil der selbstkomplementären Sequenzen umfassen.

12. Verfahren nach Anspruch 5, wobei keine der selbstkomplementären Sequenzen sich zwischen dem ersten Sondenbereich und dem zweiten Sondenbereich befinden.

13. Verfahren nach einem der vorstehenden Ansprüche, ferner umfassend ein oder mehrere der Nachfolgenden:
(e) einen Abstandhalterteil, der den ersten Sondenbereich und den zweiten Sondenbereich trennt,
(d) eine oder mehrere Armsequenzen, die eine oder mehrere der selbstkomplementären Sequenzen umfassen, wobei die eine oder die mehreren Armsequenzen die Sondenbereiche nicht umfassen,
(e) eine oder mehrere nachweisbare Markierungen, wobei die eine oder die mehreren nachweisbare Markierungen gleich oder verschieden sein können.

14. Verfahren nach Anspruch 13, wobei die Sonde einen Abstandhalterteil umfasst, der den ersten Sondenbereich und den zweiten Sondenbereich trennt und der Abstandhalterteil eine Nukleinsäure umfasst.

15. Verfahren nach Anspruch 13 oder Anspruch 14, wobei die Sonde eine oder mehrere Armsequenzen umfasst, die eine oder mehrere der selbstkomplementären Sequenzen umfassen, wobei die eine oder die mehreren der Armsequenzen die Sondenbereiche nicht umfassen und die eine oder die mehreren der selbstkomplementären Sequenzen der einen oder der mehreren Armsequenzen komplementär zu einer selbstkomplementären Sequenz sind, die sich wenigstens teilweise innerhalb eines der Sondenbereiche befindet.

16. Verfahren nach Anspruch 13 oder Anspruch 14, wobei die Sonde eine oder mehrere Armsequenzen umfasst, die eine oder mehrere der selbstkomplementären Sequenzen umfassen, wobei die eine oder die mehreren Armsequenzen die Sondenbereiche nicht umfassen und eine erste selbstkomplementäre Sequenz von der ersten oder den mehreren Armsequenz(en) komplementär zu einer zweiten selbstkomplementären Sequenz einer zweiten der einen oder der mehreren Armsequenz(en) ist.

17. Verfahren nach Anspruch 13 oder Anspruch 14, wobei die Sonde eine oder mehrere Armsequenzen umfasst, die eine oder mehrere der selbstkomplementären Sequenzen umfasst, wobei die eine oder die mehreren Armsequenzen die Sondenbereiche nicht umfassen und wenigstens eine der einen oder der mehreren selbstkomplementären Sequenzen der einen oder der mehreren Armsequenz(en) komplementär zu wenigstens einem Teil des Abstandhalterteils ist.

18. Verfahren nach einem der Ansprüche 13 bis 17, wobei die Sonde eine oder mehrere nachweisbare Markierungen umfasst, wobei die eine oder die mehreren nachweisbaren Markierungen gleich oder verschieden sein können und wobei die nachweisbare Markierung kovalent an einen Terminus der Nukleinsäuresonde gebunden ist.

19. Verfahren nach Anspruch 18 oder Anspruch 19, wobei die Sonde eine oder mehrere nachweisbare Markierungen umfasst, wobei die eine oder die mehreren nachweisbaren Markierungen gleich oder verschieden sein können und die nachweisbare Markierung kovalent an den Abstandhalterteil der Nukleinsäuresonde gebunden ist.

20. Verfahren nach Anspruch 19, wobei die Sonde eine oder mehrere nachweisbare Markierungen umfasst, wobei eine oder mehrere nachweisbare Markierungen gleich oder verschieden sein können und die nachweisbare Markierung eine Verknüpfergruppe umfasst, wobei die Verknüpfergruppe kovalent an die Nukleinsäuresonde gebunden ist und wobei, optional, die Verknüpfergruppe einen Aminoverknüpfer umfasst.

21. Verfahren nach einem der Ansprüche 13 bis 20, wobei die nachweisbare Markierung ein fluoreszierendes Molekül ist und die Nukleinsäuresonde bevorzugt keinen Fluoreszenz-Quencherteil umfasst.

22. Verfahren nach einem der Ansprüche 13 bis 21, wobei die nachweisbare Markierung eine Kugel, optional eine Latex- oder Polystyrolkugel ist.

23. Verfahren nach einem der vorstehenden Ansprüche, wobei die Nukleinsäuresonde an einem festen Träger angeheftet ist.

24. Verfahren nach einem der vorstehenden Ansprüche, wobei der erste Sondenbereich und der zweite Sondenbereich unterschiedliche Sequenzen aufweisen oder wobei der erste Sondenbereich und der zweite Sondenbereich dieselbe Sequenz aufweisen.

25. Verfahren nach einem der vorstehenden Ansprüche, wobei die Zielnukleinsäure ein Allel ist und die erste Zielsequenz und die zweite Zielsequenz Motive desselben Allels sind.

26. Verfahren nach Anspruch 1, wobei die Nukleinsäuresonde Folgendes umfasst:
(a) einen ersten Sondenbereich spezifisch für eine erste Zielsequenz; und
(b) einen zweiten Sondenbereich spezifisch für eine zweite Zielsequenz, wobei der erste Sondenbereich und der zweite Sondenbereich nicht angrenzend sind; und
(c) selbstkomplementäre Sequenzen, wobei:
in Abwesenheit einer oder beider Zielsequenz(en) die selbstkomplementären Sequenzen der Nukleinsäuresonde derart aneinander anlagern, dass die Nukleinsäuresonde eine geschlossene Konfiguration annimmt und in Anwesenheit einer oder beider Zielsequenz(en) die Hybridisierung der Nukleinsäuresonde mit einer oder beiden Zielsequenz(en) derart begünstigt ist, dass die Sonde eine offene Konfiguration annimmt.

27. Verfahren nach Anspruch 26, wobei ein oder beide Sondenbereiche wenigstens teilweise innerhalb einer Schlaufe enthalten sind, wenn sich die Nukleinsäuresonde in einer geschlossenen Konfiguration befindet, um die Hybridisierung mit Nicht-Zielsequenzen zu verhindern.

28. Verfahren nach Anspruch 26 oder Anspruch 27, wobei die Nukleinsäuresonde in der Lage ist, zwischen den Allel-Kombinationen AB/--, AB/A-, AB/-B, --/A, --/B, A-/-B zu unterscheiden, wobei A und B verschiedene Allele bezeichnen.

29. Verfahren nach einem der vorstehenden Ansprüche, wobei eine oder beide Zielsequenzen der Klasse 1-HLA-A, HLA-B, HLA-C, HLA-E, HLA-F und HLA-G oder der Klasse II-HLA-DRA, HLA-DRB1, HLA-DRB2-9, HLA-DQA1, HLA-DOB1, HLA-DPA1, HLA-DPB1, HLA-DMA, HLA-DMB, HLA-DOA und HLA-DOB angehören.

30. Nukleinsäuresonde, wie in einem der Ansprüche 26 bis 29 definiert.

31. Kit, umfassend eine Nukleinsäuresonde nach Anspruch 30, in Kombination mit Anweisungen zum Nachweis von mehr als einer Zielsequenz auf einer oder mehreren Zielnukleinsäuresequenzen unter Verwendung der Nukleinsäuresonde und optional ein oder mehrere Reagenzien zum Nachweis der einen oder der mehreren Zielsequenzen.

32. Array von Nukleinsäuresonden, umfassend eine oder mehrere einer Vielzahl von Nukleinsäuresonden nach Anspruch 30, die sich in definierten, eindeutigen Stellen an einem festen Träger befindet.

33. Array von Nukleinsäuresonden nach Anspruch 32, wobei eine oder mehrere der Vielzahl von Nukleinsäuresonden an einem festen Träger befestigt ist.

34. Array von Nukleinsäuresonden nach Anspruch 32 oder Anspruch 33, wobei eine oder mehrere der Vielzahl von Nukleinsäuresonden innerhalb einer Vertiefung des festen Trägers enthalten ist.

35. Array von Nukleinsäuresonden nach einem der Ansprüche 32 bis 34, wobei die in verschiedenen, definierten, eindeutigen Stellen an einem festen Träger befindlichen Nukleinsäuresonden einen oder mehrere verschiedene Sondenbereiche aufweisen.

36. Array von Nukleinsäuresonden nach einem der Ansprüche 32 bis 34, wobei die in verschiedenen, definierten, eindeutigen Stellen an einem festen Träger befindlichen Nukleinsäuresonden einen oder mehrere gleiche Sondenbereiche aufweisen.

37. Array von Nukleinsäuresonden nach einem der Ansprüche 32 bis 34, wobei eine oder mehrere der Vielzahl von Nukleinsäuresonden dieselben Sondenbereiche aufweist.

## Revendications

1. Procédé de détection de plus d'une séquence cible sur un acide nucléique cible, comprenant :
(a) mettre en contact une sonde d'acide nucléique comprenant :
(i) une première région de sonde spécifique d'une première séquence cible ; et
(ii) une seconde région sonde spécifique d'une seconde séquence cible, la première région de sonde et la seconde région de sonde étant non contiguës, la première séquence cible et la seconde séquence cible étant non contiguës, ou à la fois la première région de sonde et la seconde région de sonde et la première séquence cible et la seconde séquence cible étant non contiguës, et la première séquence cible et la seconde séquence cible étant chacune un allèle HLA ; et
(iii) des séquences auto-complémentaires ;
où, en présence des séquences cibles, une hybridation entre les première et seconde régions de sonde et les première et seconde séquences cibles est en compétition avec une hybridation des régions auto-complémentaires ;
avec un échantillon présumé contenir l'acide nucléique cible ; et
(b) détecter si une ou plusieurs de la première région de sonde et de la seconde région de sonde lient leur séquence cible respective, le procédé comprenant facultativement l'étape supplémentaire consistant à :
(c) amplifier les acides nucléiques dans l'échantillon présumé contenir l'acide nucléique cible avant (a).

2. Procédé selon la revendication 1, dans lequel la première séquence cible et la seconde séquence cible sont sur le même acide nucléique cible.

3. Procédé selon la revendication 1 ou la revendication 2, dans lequel la sonde d'acide nucléique est capable de distinguer un acide nucléique cible qui comprend à la fois la première séquence cible et la seconde séquence cible d'acides nucléiques qui ne comprennent pas à la fois la première séquence cible et la seconde séquence cible.

4. Procédé selon la revendication 1, dans lequel la première séquence cible et la seconde séquence cible sont sur différents acides nucléiques cibles.

5. Procédé selon l'une quelconque des revendications précédentes, dans lequel la sonde d'acide nucléique comprend deux ou quatre séquences auto-complémentaires.

6. Procédé selon l'une quelconque des revendications précédentes, dans lequel au moins l'une de la première région de sonde et de la seconde région de sonde comprend au moins une partie de l'une des séquences auto-complémentaires.

7. Procédé selon l'une quelconque des revendications 1 à 5, dans lequel la première région de sonde comprend au moins une partie de l'une des séquences auto-complémentaires et la seconde région de sonde comprend au moins une partie d'une autre des séquences auto-complémentaires.

8. Procédé selon la revendication 7, dans lequel la séquence auto-complémentaire de la première région de sonde est complémentaire à la séquence auto-complémentaire de la seconde région de sonde.

9. Procédé selon la revendication 7, dans lequel la séquence auto-complémentaire de la première région de sonde n'est pas complémentaire à la seconde région de sonde et la séquence auto-complémentaire de la seconde région de sonde n'est pas complémentaire à la première région de sonde.

10. Procédé selon la revendication 5, dans lequel seule l'une de la première région de sonde et de la seconde région de sonde comprend l'une des séquences auto-complémentaires.

11. Procédé selon la revendication 5, dans lequel ni la première région de sonde ni la seconde région de sonde ne comprennent au moins une partie des séquences auto-complémentaires.

12. Procédé selon la revendication 5, dans lequel aucune des séquences auto-complémentaires n'est située entre la première région de sonde et la seconde région de sonde.

13. Procédé selon l'une quelconque des revendications précédentes, comprenant en outre un ou plusieurs parmi :
(a) une fraction espaceur qui sépare la première région de sonde et la seconde région de sonde ;
(b) une ou plusieurs séquences de bras qui comprennent une ou plusieurs des séquences auto-complémentaires, la ou les séquences bras ne comprenant pas les régions de sonde ;
(c) un ou plusieurs marqueurs détectables, le ou les marqueurs détectables pouvant être identiques ou différents.

14. Procédé selon la revendication 13, dans lequel la sonde comprend une fraction espaceur qui sépare la première région de sonde et la seconde région de sonde et la fraction espaceur comprend un acide nucléique.

15. Procédé selon la revendication 13 ou la revendication 14, dans lequel la sonde comprend une ou plusieurs séquences de bras qui comprennent une ou plusieurs des séquences auto-complémentaires, la ou les séquences de bras ne comprenant pas les régions de sonde et la ou les séquences auto-complémentaires de la ou des séquences de bras étant complémentaires d'une séquence auto-complémentaire située au moins partiellement à l'intérieur de l'une des régions de sonde.

16. Procédé selon la revendication 13 ou la revendication 14, dans lequel la sonde comprend une ou plusieurs séquences de bras qui comprennent une ou plusieurs des séquences auto-complémentaires, la ou les séquences de bras ne comprenant pas les régions de sonde et une première séquence auto-complémentaire d'une première de la ou des séquences de bras étant complémentaire d'une seconde séquence auto-complémentaire d'une seconde de la ou des séquences de bras.

17. Procédé selon la revendication 13 ou la revendication 14, dans lequel la sonde comprend une ou plusieurs séquences de bras qui comprennent une ou plusieurs des séquences auto-complémentaires, la ou les séquences de bras ne comprenant pas les régions de sonde et au moins l'une de la ou des séquences auto-complémentaires de la ou des séquences bras étant complémentaire à au moins une partie de la fraction espaceur.

18. Procédé selon l'une des revendications 13 à 17, dans lequel la sonde comprend un ou plusieurs marqueurs détectables, le ou les marqueurs détectables pouvant être identiques ou différents, et le marqueur détectable étant lié de façon covalente à une extrémité terminale de la sonde d'acide nucléique.

19. Procédé selon la revendication 18 ou la revendication 19, dans lequel la sonde comprend un ou plusieurs marqueurs détectables, le ou les marqueurs détectables pouvant être identiques ou différents et le marqueur détectable étant lié de façon covalente à une fraction espaceur de la sonde d'acide nucléique.

20. Procédé selon la revendication 19, dans lequel la sonde comprend un ou plusieurs marqueurs détectables, le ou les marqueurs détectables pouvant être identiques ou différents et le marqueur détectable comprenant un groupe lieur, le groupe lieur étant lié de façon covalente à la sonde d'acide nucléique et facultativement le groupe lieur comprenant un lieur amino.

21. Procédé selon l'une quelconque des revendications 13 à 20, dans lequel le marqueur détectable est une molécule fluorescente et, de préférence, la sonde d'acide nucléique ne comprend pas de fraction d'extinction de fluorescence.

22. Procédé selon l'une quelconque des revendications 13 à 21, dans lequel le marqueur détectable est une perle, facultativement une perle de latex ou de polystyrène.

23. Procédé selon l'une quelconque des revendications précédentes, dans lequel la sonde d'acide nucléique est attachée à un support solide.

24. Procédé selon l'une quelconque des revendications précédentes, dans lequel la première région de sonde et la seconde région de sonde ont différentes séquences ou dans lequel la première région de sonde et la seconde région de sonde ont la même séquence.

25. Procédé selon l'une quelconque des revendications précédentes, dans lequel l'acide nucléique cible est un allèle et la première séquence cible et la seconde séquence cible sont des motifs sur le même allèle.

26. Procédé selon la revendication 1, dans lequel la sonde d'acide nucléique comprend :
(a) une première région de sonde spécifique pour une première séquence cible ; et
(b) une seconde région de sonde spécifique pour une seconde séquence cible, la première région de sonde et la seconde région de sonde n'étant pas contiguës ; et
(c) des séquences auto-complémentaires, où :
en l'absence de l'une des séquences cibles ou des deux, les séquences auto-complémentaires de la sonde d'acide nucléique s'hybrident de telle sorte que la sonde d'acide nucléique adopte une configuration fermée, et en présence de l'une des séquences cibles ou des deux une hybridation de la sonde d'acide nucléique avec l'une des séquences cibles ou les deux est favorisée de telle sorte que la sonde adopte une configuration ouverte.

27. Procédé selon la revendication 26, dans lequel l'une des ou les deux régions de sonde sont au moins partiellement contenues à l'intérieur d'une boucle lorsque la sonde d'acide nucléique est dans une configuration fermée pour empêcher une hybridation avec des séquences non cibles.

28. Procédé selon la revendication 26 ou la revendication 27, dans lequel la sonde d'acide nucléique est capable de distinguer entre des combinaisons d'allèles AB/--, AB/A-, AB/-B, --/A, --/B, A-/-B, où A et B désignent différents allèles.

29. Procédé selon l'une quelconque des revendications précédentes, dans lequel l'une des séquences cibles ou les deux sont de HLA-A, HLA-B, HLA-C, HLA-E, HLA-F et HLA-G de classe I ou HLA-DRA, HLA-DRB1, HLA-DRB2-9, HLA-DQA1, HLA-DOB1, HLA-DPA1, HLA-DPBL, HLA-DMA, HLA-DMB, HLA-DOA et HLA-DOB de classe II.

30. Sonde d'acide nucléique telle que définie à l'une quelconque des revendications 26 à 29.

31. Coffret comprenant une sonde d'acide nucléique de la revendication 30, en combinaison avec des instructions pour détecter plus d'une séquence cible sur une ou plusieurs séquences d'acide nucléique cibles à l'aide de la sonde d'acide nucléique, et facultativement un ou plusieurs réactifs pour détecter la ou les séquences cibles.

32. Réseau de sondes d'acide nucléique comprenant une ou plusieurs d'une pluralité de sondes d'acide nucléique de la revendication 30 situées dans des emplacements distincts définis sur un support solide.

33. Réseau des sondes d'acide nucléique selon la revendication 32, dans lequel une ou plusieurs de la pluralité de sondes d'acide nucléique sont attachées à un support solide.

34. Réseau de sondes d'acide nucléique selon la revendication 32 ou la revendication 33, dans lequel une ou plusieurs de la pluralité de sondes d'acide nucléique sont contenues à l'intérieur d'un puits du support solide.

35. Réseau de sondes d'acide nucléique selon l'une quelconque des revendications 32 à 34, dans lequel les sondes d'acide nucléique dans différents emplacements distincts, définis, sur le support solide, ont une ou plusieurs régions de sonde différentes.

36. Réseau de sondes d'acide nucléique selon l'une quelconque des revendications 32 à 34, dans lequel les sondes d'acide nucléique dans différents emplacements distincts, définis, sur le support solide, ont une ou plusieurs des mêmes régions de sonde.

37. Réseau de sondes d'acide nucléique selon l'une quelconque des revendications 32 à 34, dans lequel une ou plusieurs de la pluralité de sondes d'acide nucléique ont les mêmes régions de sonde.
